# EUROPEAN PATENT SPECIFICATION

(11) **EP 4 544 494 B1**
(45) Date of publication and mention of the grant of the patent: **02.12.2026**
(21) Application number: 23742585.5
(22) Date of filing: 22.06.2023
(51) Int. Cl.: G06T 7/11, G06T 7/00, G06T 7/12, G06T 7/62

(54) **OCT RETINAL VOLUMETRIC MEASUREMENTS BASED ON ETDRS GRID**
VOLUMETRISCHE OCT-RETINAMESSUNGEN AUF BASIS EINES ETDRS-GITTERS
MESURES VOLUMÉTRIQUES RÉTINIENNES OCT BASÉES SUR UNE GRILLE D'ÉTUDE ETDRS

(30) Priority: 24.06.2022 US 202263355467 P
(43) Date of publication of application: 30.04.2025
(73) Proprietor: F. Hoffmann-La Roche AG, 4070 Basel (CH)
(72) Inventor: LU, Huanxiang, 4070 Basel (CH); MAUNZ, Andreas, 4070 Basel (CH)
(74) Representative: Mewburn Ellis LLP
(86) International application number: PCT/US2023/068878
(87) International publication number: WO 2023/250417

(56) References cited:
- SCHMIDT-ERFURTH URSULA ET AL: "AI-based monitoring of retinal fluid in disease activity and under therapy", PROGRESS IN RETINAL AND EYE RESEARCH, OXFORD, GB, vol. 86, 22 June 2021 (2021-06-22), XP086924419, ISSN: 1350-9462, [retrieved on 20210622], DOI: 10.1016/J.PRETEYERES.2021.100972
- ABHIJIT GUHA ROY ET AL: "ReLayNet: Retinal Layer and Fluid Segmentation of Macular Optical Coherence Tomography using Fully Convolutional Network", ARXIV.ORG, CORNELL UNIVERSITY LIBRARY, 201 OLIN LIBRARY CORNELL UNIVERSITY ITHACA, NY 14853, 7 April 2017 (2017-04-07), XP080761372, DOI: 10.1364/BOE.8.003627
- FERRUCCI STEVEN ET AL: "Diabetic Retinopathy by the Numbers", REVIEW OF OPTOMETRY, 15 June 2016 (2016-06-15), XP093079225, Retrieved from the Internet <URL:https://www.reviewofoptometry.com/article/diabetic-retinopathy-by-the-numbers> [retrieved on 20230906]

## Description

### CROSS-REFERENCE TO RELATED APPLICATION

This application claims the benefit of U.S. Provisional Patent Application No. 63/355,467 filed June 24, 2022.

### TECHNICAL FIELD

This application relates generally to diabetic retinopathy, and, more particularly, to performing optical coherence tomography (OCT) retinal volumetric measurements based on the Early Treatment for Diabetic Retinopathy Study (ETDRS) grid.

### BACKGROUND

Diabetic retinopathy (DR) is a common complication of diabetes mellitus ("diabetes") in both Type-1 and Type-2 diabetes patients. DR may occur when high blood sugar levels cause damage to blood vessels of the retina and may include several progressive stages. For example, the stages of DR may include mild non-proliferative diabetic retinopathy (NPDR), moderate NPDR, severe NPDR, and proliferative diabetic retinopathy (PDR). Each stage of DR may include specific disease-associated features, which may occur at various locations (e.g., intraretinal, subretinal, and in the sub-retinal pigment epithelium (sub-RPE)), and may further lead to retinal detachment. For example, complications in the NPDR stages of DR may include the weakening of blood vessel walls, which may be observed as tiny bulges in the blood vessels that may leak fluids and blood into the retina. Similarly, in the PDR stage of DR, new, fragile blood vessels may form over the retina. These newly formed blood vessels may often rupture, resulting in blood leaking into the vitreous humor, the damaging of the optic nerve, or both. Left untreated, PDR may lead to severe vision loss and even blindness in diabetes patients. Furthermore, fluid leakage at any stage of DR may cause diabetic macular edema (DME) (e.g., swelling and thickening of the macula of the retina), although DME is most likely to occur during the advanced stages of DR.

DR progression, DME, and/or changes in the vasculature may be visualized utilizing, for example, color fundus photography (CFP) images or optical coherence tomography (OCT) images. For example, CFP imaging utilizes a fundus camera to record images of the interior surface of the eye to capture the retina, optic disc, macula, blood vessels of the retina, and the posterior pole (e.g., the fundus). Imaging the interior surface of the eye may allow clinicians (e.g., ophthalmologists, optometrists, or other retinal specialists) to observe the presence of DR and the potential progression of DR. OCT imaging also includes capturing the eye in a non-invasive manner utilizing light waves, reflections of which are utilized to generate a cross-sectional, two-dimensional (2D) image of the retina and retinal layers. For example, OCT imaging may distinguish retinal layers, as well as any fluids or other deposits within or around the retinal layers. OCT imaging may further depict biomarkers present in the various retinal layers, including deposits such as fatty exudates (e.g., hard, fatty deposits left by leaking blood vessels), drusen (e.g., deposits not removed due to reduced waste removal capacity), aberrant blood vessels (e.g., irregular constriction and dilatation of the vessels), blood leakage or hemorrhage, and hyperreflective material (HRMs).

Treatments for DR may vary based on the severity of the DR and/or whether DR progresses to DME. For example, clinicians (e.g., ophthalmologist, optometrist, or other retinal specialists) may generally forgo treating mild NPDR in diabetes patients, and, instead, simply observe mild NPDR over time through frequent OCT scans. In contrast, moderate NPDR, severe NPDR, and/or DME may be treated with an anti-vascular endothelial growth factor (anti-VEGF) antibody, an anti-anangiopoietin-2 (anti-Ang-2) antibody, or some combination thereof.

Visual acuity is one feature that healthcare professionals may study or test to detect the presence or progression of DR. The current standard for visual acuity testing is known as Early Treatment Diabetic Retinopathy Study (ETDRS) acuity testing. The ETDRS scale ranges from 10 (no retinopathy) to 85 (advanced PDR). ETDRS acuity testing may, in many instances, require highly-trained raters to accurately interpret 2D CFP images of a 3D structure, such as the retina. Further, CFP images may make abnormality observation challenging due to lack of depth perception. Therefore, results of the CFP images may be inaccurate, rater-dependent, and time-consuming. Schmidt-Erfurth Ursula et al. "AI-based monitoring of retinal fluid in disease activity and under therapy" (DOI: 10.1016/J.PRETEYERES.2021.100972) is a review article describing the use of Al to identify, localise and quantify retinal fluid in macular disease.

### SUMMARY

Embodiments of the present disclosure are directed toward one or more computing devices, methods, and non-transitory computer-readable media for performing optical coherence tomography (OCT) retinal volumetric measurements based on the Early Treatment for Diabetic Retinopathy Study (ETDRS) grid. In certain embodiments, the one or more computing devices may receive an optical coherence tomography (OCT) image of a retina of a patient and ETDRS mapping information identifying one or more subfields of the ETDRS grid. In certain embodiments, the one or more computing devices may segment the OCT image of the retina to identify one or more layer features corresponding to layers of the retina and one or more disease-associated features associated with the one or more layer features. In certain embodiments, the one or more computing devices may determine, based on the segmented OCT image, one or more volumetric measurements of the one or more disease-associated features, in which the one or more volumetric measurements correspond to the ETDRS mapping information. In certain embodiments, the one or more computing devices may then generate a report based on the one or more volumetric measurements.

Indeed, by generating three-dimensional (3D) volumetric measurements derivable from cross-sectional, two-dimensional (2D) OCT B-scans of a patient's retina, and by further leveraging the ETDRS grid as constructed with known dimensions and subfields and to correspond to a 2D image of a patient's fovea, the volumetric measurements (e.g., volume, thickness, area, extent, area of disruption, presence or absence of one or more disease-associated features, number of one or more disease-associated features) quantifying one or more layer features or disease-associated features of the patient's retina may be suitably mapped to 2D images (e.g., en face images, retinal thickness maps) of the patient's retina as appropriate for various clinical applications. The volumetric measurements and ETDRS mapping information may be then provided as a report, for example, to one or more the clinicians (e.g., ophthalmologists, optometrists, or other retinal specialists) for improving and facilitating the diagnosis, prognosis, and treatment of diabetic retinopathy (DR), progression of DR, and/or diabetic macular edema (DME).

In certain embodiments, the one or more layer features may include a Bruch's membrane (BM), a boundary of myoid and ellipsoid inner segments (BMEIS), a ganglion cell layer-inner plexiform layer (GCL-IPL), an inner boundary outer photoreceptor (IB-OPR) layer, an outer boundary outer photoreceptor (OB-OPR) layer, an inner boundary retinal pigment epithelium (IB-RPE) layer, an outer boundary retinal pigment epithelium (OB-RPE) layer, an internal limiting membrane (ILM), an inner plexiform layer-inner nuclear layer (IPL-INL), an inner plexiform layer-outer nuclear layer (IPL-ONL), an inner segment/outer segment junction (ISJ-OSJ) layer, outer plexiform layer-Henle's fiber layer (OPL-HFL), or an retinal nerve fiber layer-ganglion cell layer (RNFL-GCL).

In certain embodiments, the one or more computing devices the one or more disease-associated features may include one or more fluid features, in the which the one or more fluid features includes one or more of an intraretinal fluid (IRF), a subretinal fluid (SRF), or a fluid corresponding to pigment epithelial detachment (PED). In certain embodiments, the one or more disease-associated features may include one or more deposit features, in which the one or more deposit features includes a subretinal hyperreflective material (SHRM), an intraretinal hyperreflective material (IHRM), or a hyperreflective retinal foci (HRF). In certain embodiments, the one or more computing devices may identify one or more biomarkers based on the one or more volumetric measurements. In certain embodiments, the one or more computing devices may receive an en face image of the retina of the patient, in which the en face image is associated with the OCT image. In certain embodiments, prior to generating the report, the one or more computing devices may map the one or more volumetric measurements and the ETDRS mapping information to the en face image. For example, in some embodiments, mapping the one or more volumetric measurements and the ETDRS mapping information to the en face image may include associating the one or more volumetric metrics to the en face image with respect to the one or more identified subfields.

In certain embodiments, determining the one or more volumetric measurements may include determining a total volume of the one or more disease-associated features with respect to at least one of the one or more identified subfields. In certain embodiments, determining the one or more volumetric measurements may include determining a fluid volume of the one or more fluid features with respect to at least one of the one or more identified subfields. In certain embodiments, determining the one or more volumetric measurements may include determining a deposit volume of the one or more deposit features with respect to at least one of the one or more identified subfields. In certain embodiments, determining the one or more volumetric measurements may include determining a thickness of one or more of the individual layers of the retina with respect to at least one of the one or more identified subfields. In certain embodiments, determining the one or more volumetric measurements may include determining a fluid extent of the one or more fluid features with respect to at least one of the one or more identified subfields.

In certain embodiments, determining the one or more volumetric measurements may include determining a number of the one or more deposit features with respect to at least one of the one or more identified subfields. In certain embodiments, determining the one or more volumetric measurements may include determining an area of the one or more disease-associated features with respect to at least one of the one or more identified subfields. In certain embodiments, determining the one or more volumetric measurements may include determining a presence or an absence of the one or more disease-associated features with respect to at least one of the one or more identified subfields. In certain embodiments, determining the one or more volumetric measurements may include determining an area of disruption of the one or more disease-associated features with respect to at least one of the one or more identified subfields.

In certain embodiments, the one or more computing devices may classify the patient, based on the one or more volumetric measurements, as having diabetic retinopathy (DR). In certain embodiments, classifying the patient as having DR further may include classifying the patient, based on the one or more volumetric measurements, as having mild non-proliferative diabetic retinopathy (NPDR), moderate NPDR, severe NPDR, or proliferative diabetic retinopathy (PDR).

In certain embodiments, the one or more computing devices may receive a second OCT image of the retina of the patient and second ETDRS mapping information identifying one or more subfields of the ETDRS grid and segment the second OCT image of the retina to identify one or more second layer features corresponding to individual layers of the retina and one or more second disease-associated features associated with the one or more second layer features. In certain embodiments, the one or more computing devices may determine, based on the second segmented OCT image, one or more second volumetric measurements of the one or more second disease-associated features and corresponding to the second ETDRS mapping information and determine, based on the one or more second volumetric measurements, a progression of diabetic retinopathy (DR) in the patient.

In certain embodiments, the one or more computing devices may classify the patient, based on the one or more volumetric measurements, as having diabetic macula edema (DME). In certain embodiments, the one or more computing devices may generate a recommendation of a treatment for the patient based on the one or more volumetric measurements or the one or more second volumetric measurements. In certain embodiments, the treatment may include an anti-vascular endothelial growth factor (anti-VEGF) antibody, an anti-vascular endothelial growth factor-A (anti-VEGF-A) antibody, or an anti-anangiopoietin-2 (anti-Ang-2) antibody. In certain embodiments, the anti-VEGF-A antibody may include faricimab-svoa. In certain embodiments, the anti-Ang-2 antibody may include faricimab-svoa. In certain embodiments, the anti-VEGF antibody is selected from the group consisting of ranibizumab, aflibercept, brolucizumab, bevacizumab, and pegaptanib sodium. In certain embodiments, the one or more computing devices may determine, based on the one or more volumetric measurements or the one or more second volumetric measurements, whether the patient is responsive to the treatment. In certain embodiments, the one or more computing devices may identify a precision cohort associated with the patient based on the one or more volumetric measurements or the one or more second volumetric measurements. For example, in some embodiments, the precision cohort may include a group of patients identified as being clinically similar to the patient based on the one or more volumetric measurements or the one or more second volumetric measurements.

In certain embodiments, the OCT image may include a time-domain optical coherence tomography (TD-OCT) image or a spectral-domain optical coherence tomography (SD-OCT) image. In certain embodiments, the OCT image may include an image of a fovea of the patient captured by an OCT ophthalmoscope, in which the image of the fovea was further divided into three concentric circles with diameters of approximately 1 millimeter (mm), approximately 3mm, and approximately 6mm, respectively, in accordance with the ETDRS grid.

In certain embodiments, the one or more computing devices may receive an optical coherence tomography angiography (OCT-A) image of the retina of the patient and generate a retinal vascular 3D map of the retina based on the OCT-A image and the one or more volumetric measurements. In certain embodiments, the one or more computing devices may receive a color fundus photography (CFP) image of the retina of the patient and generate a composite image of the retina based on the CFP image and the one or more volumetric measurements. In certain embodiments, the report may include a table, a chart, an Extensible Markup Language (XML) file, a Hypertext Markup Language (HTML) file, a spreadsheet, a text file, an image file, a graphics file, a hyperlink, a webpage, or any combination thereof. In certain embodiments, the one or more computing devices may transmit the report to a computing device associated with a clinician. In certain embodiments, the one or more computing devices may transmit the report to an electronic device associated with the patient.

### BRIEF DESCRIPTION OF THE DRAWINGS

FIG. 1 illustrates an ophthalmic analysis and measurement system for performing OCT retinal volumetric measurements based on the ETDRS grid and generating a report based thereon.
FIG. 2A illustrates a flow diagram of a method for performing OCT retinal volumetric measurements based on the ETDRS grid and generating a report based thereon.
FIG. 2B illustrates a flow diagram of a method for performing OCT retinal volumetric measurements based on the ETDRS grid and mapping the volumetric measurements and ETDRS grid to a 2D image.
FIGs. 3, 3A, 3B, and 3C illustrate one or more high-resolution examples, respectively, for performing OCT retinal volumetric measurements based on the ETDRS grid and mapping the volumetric measurements and ETDRS grid to a thickness map.
FIGs. 4, 4A, 4B, and 4C illustrate one or more additional high-resolution examples, respectively, for performing OCT retinal volumetric measurements based on the ETDRS grid and mapping the volumetric measurements and ETDRS grid to an en face retinal image.
FIGs. 5, 5A, 5B, and 5C illustrate one or more additional high-resolution examples, respectively, for performing OCT retinal volumetric measurements based on the ETDRS grid and mapping the volumetric measurements and ETDRS grid to an en face retinal image.
FIG. 6 illustrates an example computing system for performing OCT retinal volumetric measurements based on the ETDRS grid and generating a report based thereon.

### DESCRIPTION OF EXAMPLE EMBODIMENTS

Embodiments of the present disclosure are directed toward one or more computing devices, methods, and non-transitory computer-readable media for performing optical coherence tomography (OCT) retinal volumetric measurements based on the Early Treatment for Diabetic Retinopathy Study (ETDRS) grid. In certain embodiments, the one or more computing devices may receive an optical coherence tomography (OCT) image of a retina of a patient and ETDRS mapping information identifying one or more subfields of the ETDRS grid. In certain embodiments, the one or more computing devices may segment the OCT image of the retina to identify one or more layer features corresponding to layers of the retina and one or more disease-associated features associated with the one or more layer features. In certain embodiments, the one or more computing devices may determine, based on the segmented OCT image, one or more volumetric measurements of the one or more disease-associated features, in which the one or more volumetric measurements correspond to the ETDRS mapping information. In certain embodiments, the one or more computing devices may then generate a report based on the one or more volumetric measurements.

Indeed, by generating three-dimensional (3D) volumetric measurements derivable from cross-sectional, two-dimensional (2D) OCT B-scans of a patient's retina, and by further leveraging the ETDRS grid as constructed with known dimensions and subfields and to correspond to a 2D image of a patient's fovea, the volumetric measurements (e.g., volume, thickness, area, extent, area of disruption, presence or absence of one or more disease-associated features, number of one or more disease-associated features) quantifying one or more layer features or disease-associated features of the patient's retina may be suitably mapped to 2D images (e.g., en face images, retinal thickness maps) of the patient's retina as appropriate for various clinical applications. The volumetric measurements and ETDRS mapping information may be then provided as a report, for example, to one or more the clinicians (e.g., ophthalmologists, optometrists, or other retinal specialists) for improving and facilitating the diagnosis, prognosis, and treatment of DR, progression of DR, and/or DME.

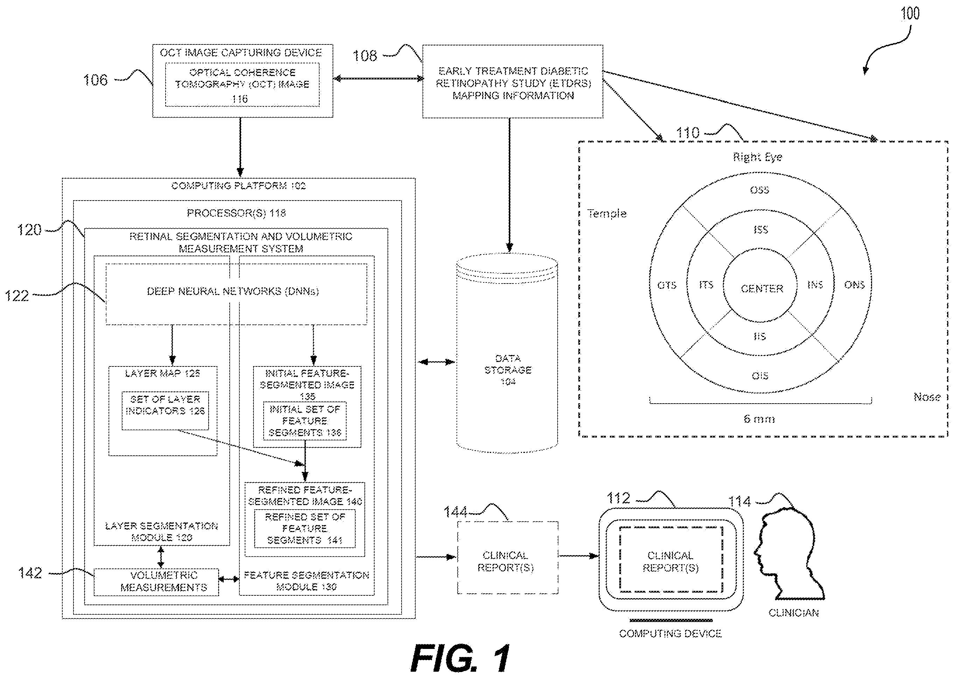

**FIG. 1** illustrates an ophthalmic analysis and measurement system 100 for performing OCT retinal volumetric measurements based on the ETDRS grid and generating a report based thereon, in accordance with the presently disclosed embodiments. The ophthalmic analysis and measurement system 100 may include a computing platform 102, a data storage 104, an OCT image-capturing device 106 (e.g., OCT ophthalmoscope), which may be associated with ETDRS mapping information 108 and ETDRS grid 110, and a computing device 112 that may be associated with one or more clinicians 114 (e.g., ophthalmologists, optometrists, or other retinal specialists) in accordance with the presently disclosed embodiments. In some embodiments, the computing platform 102 may include one or more cloud computing platforms, one or more mobile computing platforms (e.g., a smartphone, a tablet), or a combination thereof. In certain embodiments, the data storage 104, the OCT image-capturing device 106 (e.g., OCT ophthalmoscope), and the computing device 112 may be each in communication with the computing platform 102.

In certain embodiments, the OCT image-capturing device 106 (e.g., OCT ophthalmoscope) may include one or more non-invasive image-capturing devices, which may scan a patient's retina and generate one or more two-dimensional (2D), cross-sectional OCT images 116 (e.g., time-domain-OCT (TD-OCT) B-scans, spectral-domain-OCT (SD-OCT) B-scans) of a patient's retina. For example, in some embodiments, the OCT images 116 may include a number of OCT B-scans, which may be used to capture and render retinal layer depth. Specifically, in some embodiments, in capturing an image of the patient's retina, the OCT image-capturing device 106 may perform a series of one-dimensional (1D) scans (e.g., amplitude scan or "A-scan") at different depth positions and generate a 2D, cross-sectional image (e.g., brightness scan or "B-scan") of the patient's three-dimensional (3D) retina utilizing the series of A-scans.

In certain embodiments, the one or more OCT images 116 (e.g., one or more B-scans) may be associated with the ETDRS mapping information 108 and the ETDRS grid 110. For example, in accordance with the presently disclosed embodiments, the one or more OCT images 116 (e.g., one or more B-scans) may be generated along with one or more 2D images (e.g., en face image, infrared image, thickness map) each corresponding, for example, to the anatomic center of the patient's macula (e.g., the patient's fovea). Thus, the ETDRS grid 110, having been constructed to correspond to a 2D image of a patient's fovea, may be superimposed over the one or more 2D images (e.g., en face image, infrared image, thickness map) and the ETDRS mapping information 108 may identify the location of one or more features of interest or areas of interest in terms of the nine subfields of the ETDRS grid 110, including, for example: "Center" = Center point of fovea; Inner ring: ISS = "Inner Superior Subfield"; INS = "Inner Nasal Subfield"; "Inner Interior Subfield"; IIS = "Inner Inferior Subfield"; ITS = "Inner Temporal Subfield"; Outer ring: OSS = "Outer Superior Subfield"; ONS = "Outer Nasal Subfield"; OIS = "Outer Inferior Subfield"; OTS = "Outer Temporal Subfield."

In certain embodiments, the ETDRS mapping information 108 and the ETDRS grid 110 may be utilized to determine one or more volumetric measurements (e.g., volume, thickness, area, extent, area of disruption, presence or absence of one or more disease-associated features, a number of disease-associated features) for qualifying and quantifying diabetic retinopathy (DR) or other disease-associated features with respect to the anatomic center of the patient's macula (e.g., the patient's fovea) from the one or more OCT images 116 (e.g., one or more B-scans). For example, as noted above, and as will be discussed in further detail below, the one or more OCT images 116 (e.g., one or more B-scans) may be generated along with one or more 2D images (e.g., en face image, infrared image, thickness map) each corresponding, for example, to the anatomic center of the patient's macula (e.g., the patient's fovea).

In certain embodiments, once the one or more OCT images 116 (e.g., one or more B-scans) are segmented and annotated to identify the retinal layers of the patient's retina and any disease-associated features (e.g., fluids, deposit materials), the computing platform 102 may determine one or more volumetric measurements (e.g., volume, thickness, area, extent, area of disruption, presence or absence of one or more disease-associated features, a number of disease-associated features) of the retinal layers and the disease-associated features (e.g., fluids, deposit materials). Further, because the one or more OCT images 116 (e.g., one or more B-scans) and the one or more 2D images (e.g., en face image, infrared image, thickness map) may be each known to correspond to the patient's fovea, the one or more volumetric measurements determined from the one or more OCT images 116 (e.g., one or more B-scans) may be then suitably mapped to the one or more 2D images (e.g., en face image, infrared image, thickness map) in accordance with the ETDRS mapping information 108 and the ETDRS grid 110.

In certain embodiments, as further depicted by FIG. 1, the ETDRS grid 110 may bounded by a circular area with a diameter of 6 millimeters (mm). The center point of the ETDRS grid 110 may be the center of the circle. The ETDRS grid 110 may be divided into nine standard subfields. The center subfield may be a circle with a diameter of 1 mm. The ETDRS grid 110 may be further divided into four inner and four outer subfields by a circle concentric to the center with a diameter of 3 mm. The inner and outer subfields may be each divided by four radial lines extending from the center circle to the outermost circle at, for example, 45°, 135°, 225°, and 315° and transecting the 3 mm circle in four places.

In certain embodiments, each of the four inner and four outer subfields may be labeled by their orientation with respect to position relative to the center of the patient's macula: "superior", "nasal", "inferior", and "temporal". For example, in some embodiments, the superior inner subfield may be the region bounded by the center circle, the 3 mm circle, the 315° radial line, and the 45° radial line. The nasal subfields may be those oriented toward the midline of the patient's face, for example, and nearest to the optic nerve head. In some embodiments, the ETDRS grid 110 for the left and right eyes may be reversed with respect to the positions of the nasal and temporal subfields.

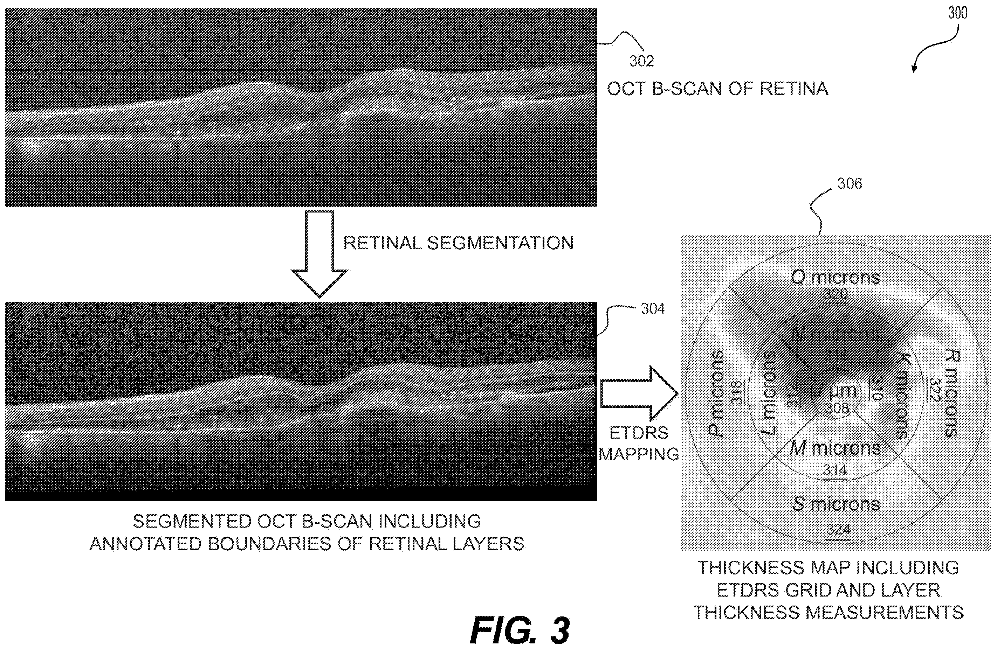

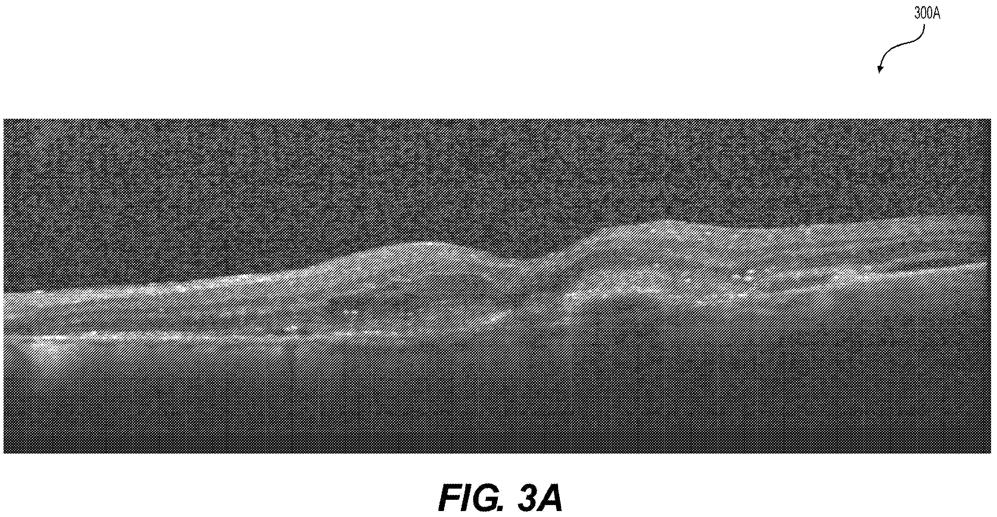

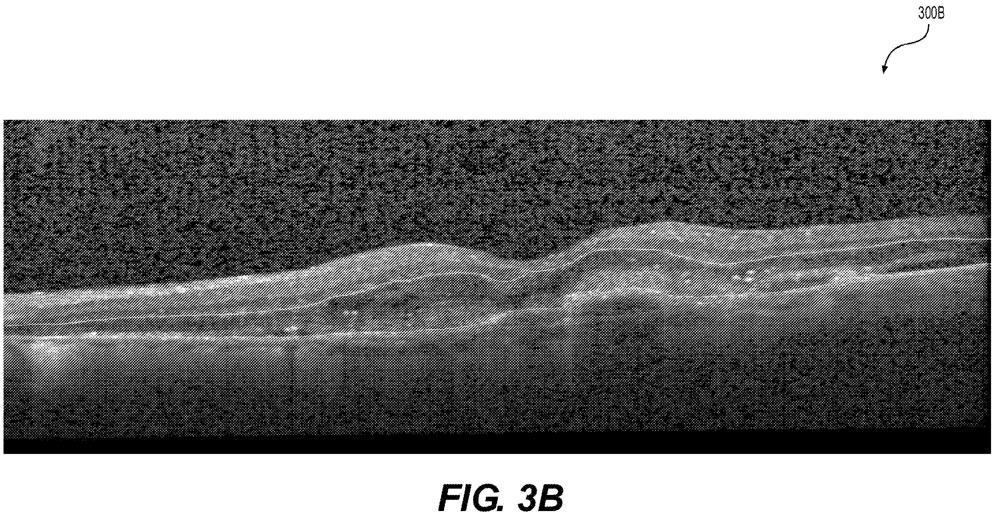

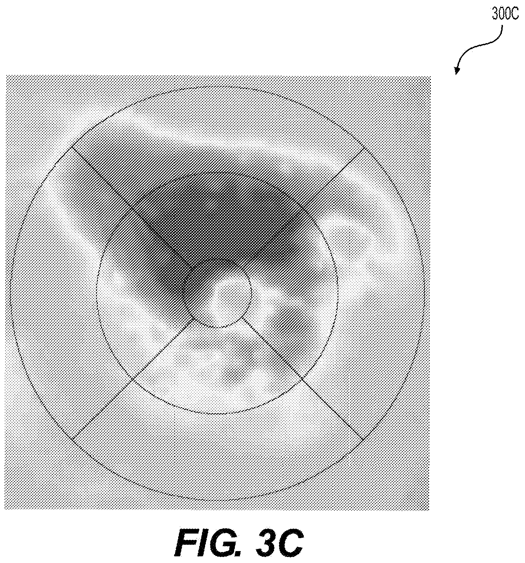

In certain embodiments, as previously noted, the ETDRS mapping information 108 may include, for example, information identifying and/or quantifying one or more features of interest or areas of interest within one or more 2D images (e.g., en face image, infrared image, thickness map) associated with the one or more OCT images 116 (e.g., one or more B-scans) in terms of the nine subfields of the ETDRS grid 110 (e.g., "Center" = Center point of fovea; Inner ring: ISS = "Inner Superior Subfield"; INS = "Inner Nasal Subfield"; "Inner Interior Subfield"; IIS = "Inner Inferior Subfield"; ITS = "Inner Temporal Subfield"; Outer ring: OSS = "Outer Superior Subfield"; ONS = "Outer Nasal Subfield"; OIS = "Outer Inferior Subfield"; OTS = "Outer Temporal Subfield."). For example, as will be further appreciated with respect to FIGs. 3, 4, and 5, the ETDRS mapping information 108 may identify locations of one or more features of interest or areas of interest in terms of the ETDRS grid 110 (e.g., within one or more of the nine individual subfields, within the outer ring including the OSS, ONS, OIS, and OTS subfields, within the inner ring including the ISS, INS, IIS, and ITS subfields, discs or partial discs including the Center, ITS, and OTS subfields, discs or partial discs including the Center, ISS, and OSS subfields, discs or partial discs including the Center, INS, and ONS subfields, discs or partial discs including the Center, ITS, and OTS subfields, or any of various combinations of thereof).

In certain embodiments, the ophthalmic analysis and measurement system 100 may include one or more processor(s) 118, which may be implemented using hardware, software, firmware, or a combination thereof. In some embodiments, the one or more processor(s) 118 may be included as part of the computing platform 102, and may be further utilized, for example, to support a retinal segmentation and volumetric measurement system 120 utilized to segment and annotate the one or more OCT images 116 (e.g., one or more B-scans) to label one or more of the layers of the patient's retina, one or more fluids associated with one or more of the layers of the patient's retina, or one or more materials associated with one or more of the layers of the patient's retina. In certain embodiments, the retinal segmentation and volumetric measurement system 120 may include one or more deep neural networks (DNNs) 122 or other similar machine-learning models suitable for performing image segmentation (e.g., semantic image segmentation), feature extraction and selection, and classification of the one or more OCT images 116 (e.g., one or more B-scans).

In certain embodiments, the one or more deep-learning models 122 may include, for example, a deep residual neural network (ResNet) image-classification network (e.g., ResNet-50, ResNet-101, ResNet-152), a full-resolution residual network (FRRN), a fully convolutional network (FCN) (e.g., U-Net), a pyramid scene parsing network (PSPNet), a fully convolutional dense neural network (FCDenseNet), a multi-path refinement network (RefineNet), an atrous convolutional network (e.g., DeepLabV3, DeepLabV+), a semantic segmentation network (SegNet), or other deep convolutional network (DCNN) that may be suitable for performing semantic segmentation and feature extraction and selection to segment and annotate one or more layer features (e.g., layers of the retina) and one or more fluidic features or deposit features detectable from the OCT images 116 (e.g., one or more B-scans). For example, in certain embodiments, the retinal segmentation and volumetric measurement system 120 may receive the one or more OCT images 116 (e.g., one or more B-scans) for processing. In some embodiments, the one or more OCT images 116 may include an image of a patient's retina, which includes a diabetes-related eye disease, such as DR or DME.

In certain embodiments, as further depicted, the retinal segmentation and volumetric measurement system 120 may include layer identification module 120 and feature segmentation module 130, each of which may be implemented using software, firmware, hardware, or a combination thereof. In certain embodiments, the layer identification module 120 and the feature segmentation module 130 may, in conjunction, be utilized to annotate the one or more OCT images 116 (e.g., one or more B-scans) to assign class labels to one or more of the layers of the retina and disease-associated features (e.g., fluids, reflective materials) that may be associated with the layers of the retina.

For example, in certain embodiments, the layer identification module 120 may generate layer map 125 that includes set of layer indicators 126. The set of layer indicators 126 identify one or more retinal layers. In some embodiments, the set of layer indicators 126 may include one or more layer features, in which each layer feature may correspond, for example, to one or more the boundaries (e.g., inner boundary and/or outer boundary) of a corresponding retinal layer of the retina. For example, in some embodiments, the one or more layer features may be a Bruch's membrane (BM), a boundary of myoid and ellipsoid inner segments (BMEIS), a ganglion cell layer-inner plexiform layer (GCL-IPL), an inner boundary outer photoreceptor (IB-OPR) layer, an outer boundary outer photoreceptor (OB-OPR) layer, an inner boundary retinal pigment epithelium (IB-RPE) layer, an outer boundary retinal pigment epithelium (OB-RPE) layer, an internal limiting membrane (ILM), an inner plexiform layer-inner nuclear layer (IPL-INL), an inner plexiform layer-outer nuclear layer (IPL-ONL), an inner segment/outer segment junction (ISJ-OSJ) layer, outer plexiform layer-Henle's fiber layer (OPL-HFL), or an retinal nerve fiber layer-ganglion cell layer (RNFL-GCL).

In certain embodiments, the set of layer indicators 126 may include a set of layer segments, in which each layer segment is a region that identifies a thickness of a corresponding retinal layer. In one embodiment, a layer segment may be a continuous or discontinuous region. In certain embodiments, the feature segmentation module 130 may generate an initial feature-segmented image 135 that includes initial sets of feature segments 136. In certain embodiments, the initial feature-segmented image 135 including the initial sets of feature segments 136 and the layer map 125 including the set of layer indicators 126 may be combined, for example, into a refined feature-segmented image 140 including refined sets of feature segments 141. In certain embodiments, the refined feature-segmented image 140 and the refined sets of feature segments 141 may include the one or more OCT images 116 (e.g., one or more B-scans) including annotations of one or more retinal layers and a set of disease-associated features (e.g., fluids, deposit materials). For example, the one or more disease-associated features may include fluid features, which may include one or more of an intraretinal fluid (IRF), a subretinal fluid (SRF), or a fluid corresponding to pigment epithelial detachment (PED). Additionally, the one or more disease-associated features may include deposit materials, which may include a subretinal hyperreflective material (SHRM), an intraretinal hyperreflective material (IHRM), or a hyperreflective retinal foci (HRF).

In certain embodiments, after the one or more OCT images 116 (e.g., one or more B-scans) are segmented and annotated to include class labels for the layers of the retina and the disease-associated features (e.g., fluids, deposit materials), the computing platform 102 may then determine one or more volumetric measurements 142 (e.g., volume measurements, thickness measurements, area measurements, extent measurements, area of disruption measurements) from the one or more OCT images 116 (e.g., one or more B-scans). Specifically, in certain embodiments, once the one or more OCT images 116 (e.g., one or more B-scans) are segmented and annotated to identify the retinal layers of the patient's retina and any disease-associated features (e.g., fluids, deposit materials), the computing platform 102 may determine the one or more volumetric measurements 142 (e.g., volume, thickness, area, extent, area of disruption, presence or absence of one or more disease-associated features, a number of one or more disease-associated features) of the retinal layers and the disease-associated features (e.g., fluids, deposit materials) from the one or more OCT images 116 (e.g., one or more B-scans).

For example, in some embodiments, the computing platform 102 may determine the one or more volumetric measurements 142 (e.g., volume, thickness, area, extent, area of disruption, presence or absence of one or more disease-associated features, a number of one or more disease-associated features) from the one or more OCT images 116 (e.g., one or more B-scans) based on the ETDRS mapping information 108 and the ETDRS grid 110. As previously noted, the one or more OCT images 116 (e.g., one or more B-scans) may each be captured and generated along with one or more 2D images (e.g., en face image, infrared image, thickness map), in which the one or more OCT images 116 (e.g., one or more B-scans) and the one or more 2D images (e.g., en face image, infrared image, thickness map) may be each known to correspond to the patient's fovea.

Thus, in certain embodiments, based on the ETDRS mapping information 108 and the known measurements and dimensions (e.g., three concentric circles with diameters of 1 mm, 3mm, and 6mm, respectively, and the four radial lines extending from the center circle to the outermost circle and transecting the 3 mm circle in four places) of the ETDRS grid 110, as well as the knowledge of the one or more OCT images 116 (e.g., one or more B-scans) with respect to depth information (e.g., retinal layer depth), the computing platform 102 may utilize one or more image processing techniques (e.g., morphological image processing) to estimate the one or more volumetric measurements 142. For example, in some embodiments, the computing platform 102 may derive a number of subspace constraints (e.g., an indication of the 3D subspaces, such as intraretinal subspaces and subretinal subspaces in which the disease-associated features may occur) based on the layer features (e.g., BM, BMEIS, GCL-IPL, IB-OPR, OB-OPR layer, IB-RPE, OB-RPE, ILM, IPL-INL, IPL-ONL, ISJ-OSJ, OPL-HFL, RNFL-GCL), and the derived number of subspace constraints may be then mapped and/or masked with respect to the nine subfields of the ETDRS grid 110. The computing platform 102 may then determine the one or more volumetric measurements 142 (e.g., volume, thickness, area, extent, area of disruption, presence or absence of one or more disease-associated features, a number of one or more disease-associated features) by, for example, counting the numbers of pixels per subfield of the ETDRS grid 110 and converting the determined numbers of pixels per subfield of the ETDRS grid 110 to microns for thicknesses, squared microns for areas, and cubic microns for volumes, and so forth.

In certain embodiments, the volumetric measurements 142 may include, for example, a total volume of the one or more the disease-associated features (e.g., a total volume in cubic microns between various layers of the retina) with respect to one or more of the nine subfields of the ETDRS grid 110, a fluid volume of the one or more fluid features (e.g., a total volume in cubic microns for various fluids) with respect to one or more of the nine subfields of the ETDRS grid 110, a deposit volume of the one or more deposit features (e.g., a total volume in cubic microns for various deposit materials) with respect to one or more of the nine subfields of the ETDRS grid 110, a thickness of one or more of the layers of the retina (e.g., thickness measurements in microns of all of the layers of the retina, thickness measurements in microns with respect to one or more specific layers of the retina, or thickness measurements in microns of the "slabs" or spaces between the layers of the retina) with respect to one or more of the nine subfields of the ETDRS grid 110, a fluid extent of the one or more fluid features (e.g., volume measurements in microns for various fluids) with respect to one or more of the nine subfields of the ETDRS grid 110, or a number of the one or more deposit features (e.g., a numerical value representing the total number of identified deposit materials) with respect to one or more of the nine subfields of the ETDRS grid 110.

In certain embodiments, the one or more volumetric measurements 142 may further include, for example, an area of the one or more disease-associated features (e.g., en face image area measurements in squared microns for various fluids or deposit materials) with respect to one or more of the nine subfields of the ETDRS grid 110, an indication of a presence or an absence of the one or more disease-associated features (e.g., a binary value representing either a presence or an absence of various fluids and deposit materials) with respect to one or more of the nine subfields of the ETDRS grid 110, an area of disruption of the one or more disease-associated features (e.g., en face image area of disruption in squared microns for various fluids or deposit materials) with respect to one or more of the nine subfields of the ETDRS grid 110, or one or more other volumetric measurements that may be utilized, for example, by one or more the clinicians 114 (e.g., ophthalmologists, optometrists, or other retinal specialists) to qualify and quantify DR-associated features, DME-associated features, or other disease-associated features with respect to the anatomic center of the patient's macula (e.g., the patient's fovea).

In some embodiments, as further illustrated by FIG. 1, after the one or more volumetric measurements 142 are determined, the one or more volumetric measurements 142 and the corresponding one or more segmented and annotated OCT images 116 (e.g., one or more B-scans) may be stored to the data storage 104 by the computing platform 102. In other embodiments, as further illustrated by FIG. 1, after the one or more volumetric measurements 142 are determined, the one or more volumetric measurements 142 and the corresponding one or more segmented and annotated OCT images 116 (e.g., one or more B-scans) may be included in one or more clinical reports 144 and then transmitted by the computing platform 102 to the computing device 112 associated with one or more the clinicians 114 (e.g., ophthalmologists, optometrists, or other retinal specialists) to be analyzed and examined.

For example, in some embodiments, the clinical report 144 may include, for example, a table, a chart, an Extensible Markup Language (XML) file, a Hypertext Markup Language (HTML) file, a spreadsheet, a text file, an image file, a graphics file, a hyperlink, a webpage, or other file that may be accessible and viewable on the computing device 112 by the one or more the clinicians 114 (e.g., ophthalmologists, optometrists, or other retinal specialists). In one embodiment, the one or more clinical reports 144 may also be transmitted by the computing platform 102 to a computing device associated with a patient or one or more additional scientific or medical professionals (e.g., biomarker scientists, data scientists) for further analysis and/or clinical application.

In certain embodiments, because the one or more volumetric measurements 142 may be associated with the ETDRS mapping information 108 and the ETDRS grid 110, the one or more clinicians 114 (e.g., ophthalmologists, optometrists, or other retinal specialists) may utilize the clinical report 144 to accurately and efficiently qualify and quantify DR-associated features, DME-associated features, or other disease-associated features with respect to the anatomic center of the patient's macula (e.g., the patient's fovea). For example, in one embodiment, as will be further appreciated with respect to examples 300, 400, and 500 illustrated by FIGs. 3, 4, and 5, respectively, the clinical report 144 may include retinal volumetric measurements for each of the nine subfields of the ETDRS grid 110. In certain embodiments, the one or more clinicians 114 (e.g., ophthalmologists, optometrists, or other retinal specialists) may then utilize the retinal volumetric measurements to determine, for example, whether one or more of the retinal volumetric measurements fall outside the normative range to determine a diagnosis of DR and/or DME, one or more treatments for DR and/or DME, and/or a progression of DR and/or DME.

In certain embodiments, based on the one or more volumetric measurements 142, the computing platform 102 may classify the patient as having DR and/or DME and generate a recommendation of one or more suitable treatments (e.g., an anti-vascular endothelial growth factor (anti-VEGF) antibody, an anti-vascular endothelial growth factor-A (anti-VEGF-A) antibody, or an anti-anangiopoietin-2 (anti-Ang-2) antibody) for the patient and include these data within the clinical report 144 to be provided to the one or more the clinicians 114 (e.g., ophthalmologists, optometrists, or other retinal specialists). For example, in some embodiments, the anti-VEGF-A antibody may include faricimab-svoa and the anti-Ang-2 antibody may include faricimab-svoa. In some embodiments, the anti-VEGF antibody may be selected from the group consisting of ranibizumab, aflibercept, brolucizumab, bevacizumab, and pegaptanib sodium.

In certain embodiments, the computing platform 102 may identify a precision cohort associated with the patient based on the one or more volumetric measurements 142. For example, in some embodiments, the precision cohort may include a group of patients identified as being clinically similar to the patient based on the one or more volumetric measurements 142. For example, in one embodiment, the precision cohort may be identified as having a same stage of DR or other similar retinal disease as the patient and/or as responding best to one or more particular treatment regimens, such that the patient may be recommended to undergo the same or a similar treatment regimen as the precision cohort.

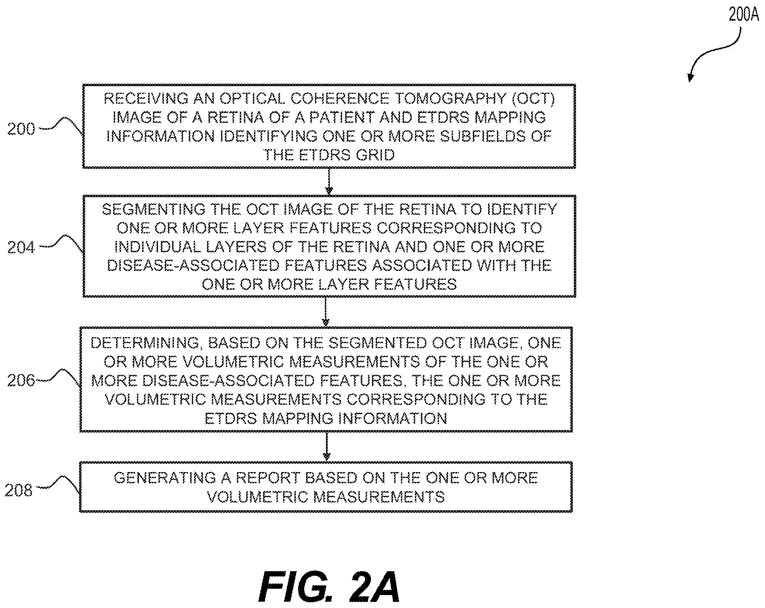

**FIG. 2A** illustrates a flow diagram of a method 200A for performing OCT retinal volumetric measurements based on the ETDRS grid and generating a report based thereon, in accordance with the disclosed embodiments. The method 200A may be performed utilizing one or more processing devices (e.g., computing platform 102 as discussed above with respect to FIG. 1) that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), or any other processing device(s) that may be suitable for processing retinal data and making one or more decisions based thereon), firmware (e.g., microcode), or some combination thereof.

The method 200A may begin at block 202 with the one or more processing devices receiving an optical coherence tomography (OCT) image of a retina of a patient and ETDRS mapping information identifying one or more subfields of the ETDRS grid. For example, in some embodiments, the computing platform 102 may receive one or more OCT B-scans of the patient's retina. The method 200A may then continue at block 204 with the one or more processing devices segmenting the OCT image of the retina to identify one or more layer features corresponding to layers of the retina and one or more disease-associated features associated with the one or more layer features. For example, in some embodiments, the computing platform 102 may segment the OCT images 116 (e.g., one or more B-scans) and identify and annotate one or more retinal layers and a set of disease-associated features (e.g., fluids, deposit materials). The one or more disease-associated features may include, for example, fluid features (e.g., IRF, SRF, PED, and so forth) and deposit materials (e.g., SHRM, IHRM, HRF, and so forth).

The method 200A may then continue at block 206 with the one or more processing devices determining, based on the segmented OCT image, one or more volumetric measurements of the one or more disease-associated features, in which the one or more volumetric measurements correspond to the ETDRS mapping information. For example, as previously discussed above with respect to FIG. 1, based on the ETDRS mapping information 108 and the known measurements and dimensions (e.g., three concentric circles with diameters of 1 mm, 3mm, and 6mm, respectively, and the four radial lines extending from the center circle to the outermost circle and transecting the 3 mm circle in four places) of the ETDRS grid 110, as well as the knowledge of the one or more OCT images 116 (e.g., one or more B-scans) with respect to depth information (e.g., retinal layer depth), the computing platform 102 may utilize one or more image processing techniques (e.g., morphological image processing) to determine a number of volumetric measurements 142.

For example, in some embodiments, the computing platform 102 may derive a number of subspace constraints (e.g., an indication of the 3D subspaces, such as intraretinal subspaces and subretinal subspaces in which the disease-associated features may occur) based on the layer features (e.g., BM, BMEIS, GCL-IPL, IB-OPR, OB-OPR layer, IB-RPE, OB-RPE, ILM, IPL-INL, IPL-ONL, ISJ-OSJ, OPL-HFL, RNFL-GCL), and the derived number of subspace constraints may be then mapped and/or masked with respect to the nine subfields of the ETDRS grid 110. The computing platform 102 may then determine the one or more volumetric measurements 142 (e.g., volume, thickness, area, extent, area of disruption, presence or absence of one or more disease-associated features, a number of one or more disease-associated features) by, for example, counting the numbers of pixels per subfield of the ETDRS grid 110 and converting the determined numbers of pixels per subfield of the ETDRS grid 110 to microns for thicknesses, squared microns for areas, and cubic microns for volumes, and so forth.

For example, the number of volumetric measurements 142 may include, for example, a total volume of the one or more the disease-associated features (e.g., a total volume in cubic microns between various layers of the retina) with respect to one or more of the nine subfields of the ETDRS grid 110, a fluid volume of the one or more fluid features (e.g., a total volume in cubic microns for various fluids) with respect to one or more of the nine subfields of the ETDRS grid 110, a deposit volume of the one or more deposit features (e.g., a total volume in cubic microns for various deposit materials) with respect to one or more of the nine subfields of the ETDRS grid 110, a thickness of one or more of the layers of the retina (e.g., thickness measurements in microns of all of the layers of the retina, thickness measurements in microns with respect to one or more specific layers of the retina, or thickness measurements in microns of the "slabs" or spaces between the layers of the retina) with respect to one or more of the nine subfields of the ETDRS grid 110, a fluid extent of the one or more fluid features (e.g., volume measurements in microns for various fluids) with respect to one or more of the nine subfields of the ETDRS grid 110, or a number of the one or more deposit features (e.g., a numerical value representing the total number of identified deposit materials) with respect to one or more of the nine subfields of the ETDRS grid 110.

In certain embodiments, the number of volumetric measurements 142 may further include, for example, an area of the one or more disease-associated features (e.g., en face image area measurements in squared microns for various fluids or deposit materials) with respect to one or more of the nine subfields of the ETDRS grid 110, an indication of a presence or an absence of the one or more disease-associated features (e.g., a binary value representing either a presence or an absence of various fluids and deposit materials) with respect to one or more of the nine subfields of the ETDRS grid 110, or an area of disruption of the one or more disease-associated features (e.g., en face image area of disruption in squared microns for various fluids or deposit materials) with respect to one or more of the nine subfields of the ETDRS grid 110. The method 200A may then conclude at block 208 with one or more processing devices determining generating a report based on the one or more volumetric measurements. For example, as generally discussed above, the clinical report 144 may include, for example, a table, a chart, an XML file, a HTML file, a spreadsheet, a text file, an image file, a graphics file, a hyperlink, a webpage, or other file that may be accessible and viewable on the computing device 112 by the one or more the clinicians 114 (e.g., ophthalmologists, optometrists, or other retinal specialists).

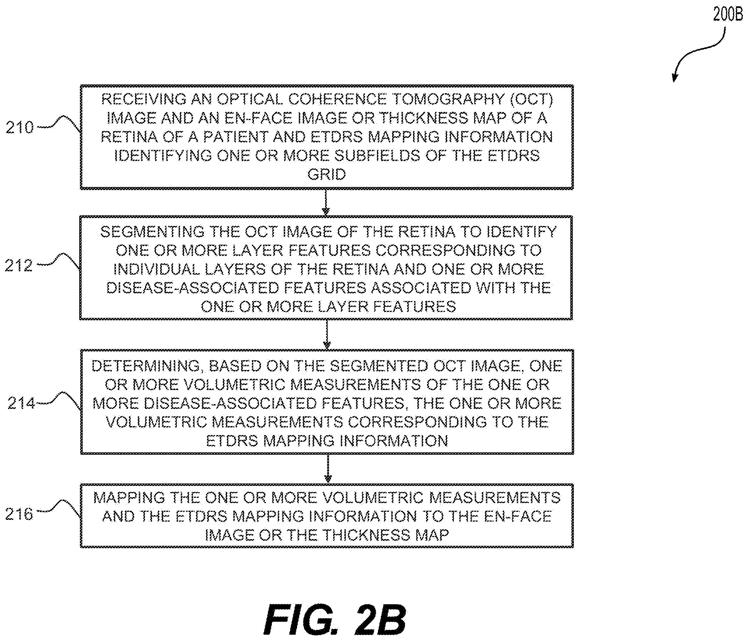

**FIG. 2B** illustrates a flow diagram of a method 200B for performing OCT retinal volumetric measurements based on the ETDRS grid and mapping the volumetric measurements and ETDRS grid to a 2D retinal image, in accordance with the disclosed embodiments. The method 200B may be performed utilizing one or more processing devices (e.g., computing platform 102 as discussed above with respect to FIG. 1) that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), or any other processing device(s) that may be suitable for processing retinal data and making one or more decisions based thereon), firmware (e.g., microcode), or some combination thereof.

The method 200B may begin at block 210 with the one or more processing devices receiving an optical coherence tomography (OCT) image and en face image or thickness map of a retina of a patient and ETDRS mapping information identifying one or more subfields of the ETDRS grid. For example, in some embodiments, the computing platform 102 may receive one or more OCT images 116 (e.g., one or more B-scans) and one or more 2D images (e.g., en face image, infrared image, thickness map) each known to correspond to the patient's fovea. The method 200B may then continue at block 212 with the one or more processing devices segmenting the OCT image of the retina to identify one or more layer features corresponding to layers of the retina and one or more disease-associated features associated with the one or more layer features. For example, in some embodiments, the computing platform 102 may segment the OCT images 116 (e.g., one or more B-scans) and identify and annotate one or more retinal layers and a set of disease-associated features (e.g., fluids, deposit materials). The one or more disease-associated features may include, for example, fluid features (e.g., IRF, SRF, PED, and so forth) and deposit materials (e.g., SHRM, IHRM, HRF, and so forth).

The method 200B may then continue at block 214 with the one or more processing devices determining, based on the segmented OCT image, one or more volumetric measurements of the one or more disease-associated features, in which the one or more volumetric measurements correspond to the ETDRS mapping information. For example, as previously discussed above with respect to FIG. 1, based on the ETDRS mapping information 108 and the known measurements and dimensions (e.g., three concentric circles with diameters of 1 mm, 3mm, and 6mm, respectively, and the four radial lines extending from the center circle to the outermost circle and transecting the 3 mm circle in four places) of the ETDRS grid 110, as well as the knowledge of the one or more OCT images 116 (e.g., one or more B-scans) with respect to depth information (e.g., retinal layer depth), the computing platform 102 may utilize one or more image processing techniques (e.g., morphological image processing) to determine a number of volumetric measurements 142.

For example, in some embodiments, the computing platform 102 may derive a number of subspace constraints (e.g., an indication of the 3D subspaces, such as intraretinal subspaces and subretinal subspaces in which the disease-associated features may occur) based on the layer features (e.g., BM, BMEIS, GCL-IPL, IB-OPR, OB-OPR layer, IB-RPE, OB-RPE, ILM, IPL-INL, IPL-ONL, ISJ-OSJ, OPL-HFL, RNFL-GCL), and the derived number of subspace constraints may be then mapped and/or masked with respect to the nine subfields of the ETDRS grid 110. The computing platform 102 may then determine the one or more volumetric measurements 142 (e.g., volume, thickness, area, extent, area of disruption, presence or absence of one or more disease-associated features, a number of one or more disease-associated features) by, for example, counting the numbers of pixels per subfield of the ETDRS grid 110 and converting the determined numbers of pixels per subfield of the ETDRS grid 110 to microns for thicknesses, squared microns for areas, and cubic microns for volumes, and so forth.

In certain embodiments, the number of volumetric measurements 142 may include, for example, a total volume of the one or more the disease-associated features (e.g., a total volume in cubic microns between various layers of the retina) with respect to one or more of the nine subfields of the ETDRS grid 110, a fluid volume of the one or more fluid features (e.g., a total volume in cubic microns for various fluids) with respect to one or more of the nine subfields of the ETDRS grid 110, a deposit volume of the one or more deposit features (e.g., a total volume in cubic microns for various deposit materials) with respect to one or more of the nine subfields of the ETDRS grid 110, a thickness of one or more of the layers of the retina (e.g., thickness measurements in microns of all of the layers of the retina, thickness measurements in microns with respect to one or more specific layers of the retina, or thickness measurements in microns of the "slabs" or spaces between the layers of the retina) with respect to one or more of the nine subfields of the ETDRS grid 110, a fluid extent of the one or more fluid features (e.g., volume measurements in microns for various fluids) with respect to one or more of the nine subfields of the ETDRS grid 110, or a number of the one or more deposit features (e.g., a numerical value representing the total number of identified deposit materials) with respect to one or more of the nine subfields of the ETDRS grid 110.

In certain embodiments, the one or more volumetric measurements 142 may further include, for example, an area of the one or more disease-associated features (e.g., en face image area measurements in squared microns for various fluids or deposit materials) with respect to one or more of the nine subfields of the ETDRS grid 110, an indication of a presence or an absence of the one or more disease-associated features (e.g., a binary value representing either a presence or an absence of various fluids and deposit materials) with respect to one or more of the nine subfields of the ETDRS grid 110, or an area of disruption of the one or more disease-associated features (e.g., en face image area of disruption in squared microns for various fluids or deposit materials) with respect to one or more of the nine subfields of the ETDRS grid 110.

The method 200B may then conclude at block 216 with the one or more processing devices mapping the one or more volumetric measurements and the ETDRS mapping information to the en face image or the thickness map. For example, as previously noted, once the one or more OCT images 116 (e.g., one or more B-scans) are segmented and annotated to identify the retinal layers of the patient's retina and any disease-associated features (e.g., fluids, deposit materials), the computing platform 102 may determine one or more volumetric measurements (e.g., volume, thickness, area, extent, area of disruption, presence or absence of one or more disease-associated features) of the retinal layers and the disease-associated features (e.g., fluids, deposit materials). For example, because the one or more OCT images 116 (e.g., one or more B-scans) and the en face image or thickness map may be each known to correspond to the patient's fovea, the one or more volumetric measurements 142 determined from the one or more OCT images 116 (e.g., one or more B-scans) may be suitably mapped to the en face image or thickness map in accordance with the ETDRS mapping information 108 and the ETDRS grid 110.

**FIGs. 3****,** **3A****,** **3B****,** and **3C** illustrate one or more high-resolution examples 300, 300A, 300B, and 300C, respectively, for performing OCT retinal volumetric measurements based on the ETDRS grid and mapping the volumetric measurements and ETDRS grid to a thickness map, in accordance with the disclosed embodiments. It should be appreciated that the one or more high-resolution examples 300, 300A, 300B, and 300C may represent only one example of the presently disclosed embodiments. Indeed, in other examples, in accordance with the presently disclosed embodiments, any of various retinal volumetric measurements (e.g., volume measurements, thickness measurements, area measurements, extent measurements, presence or absence indications, area of disruption measurements, and so forth) may be computed for each OCT B-scan and for any of the various subfields of the ETDRS grid.

For example, the high-resolution example 300 of FIG. 3 illustrates an OCT B-scan 302 (e.g., corresponding to high-resolution OCT B-scan 300A of FIG. 3A), a segmented and annotated OCT B-scan 304 (e.g., corresponding to high-resolution segmented and annotated OCT B-scan 300B of FIG. 3B), and a thickness map 306 (e.g., corresponding to high-resolution thickness map 300C of FIG. 3C) illustrating one or more layer thickness measurements. For example, referring to FIG. 3, the OCT B-scan 302 may be an OCT B-scan generated based on one or more scans of patient's retina by an ophthalmoscope or other retinal image-capturing device. The OCT B-scan 302 may be associated with the ETDRS grid and/or ETDRS mapping information (e.g., location of one or more features or areas of interest in terms of the nine subfields of ETDRS grid). Similarly, the thickness map 306 may be generated together with the OCT B-scan 302, and the OCT B-scan 302 and the thickness map 306 may each correspond, for example, to images of the anatomic center of a patient's macula (e.g., the patient's fovea).

As further illustrated, the segmented and annotated OCT B-scan 304 may include annotations (e.g., colored lines) labeling one or more boundaries of the layers of the patient's retina. One or more retinal volumetric measurements (e.g., thickness of each of the layer features of the retina) may be then determined based on the ETDRS grid and/or ETDRS mapping information, and the one or more retinal volumetric measurements may be then mapped to the thickness map 306 corresponding to the OCT B-scan 302. Specifically, because the OCT B-scan 302 and the thickness map 306 may be each known to correspond to the patient's fovea, the one or more volumetric measurements determined from the segmented and annotated OCT B-scan 304 may be suitably mapped to the thickness map 306 in accordance with the ETDRS grid and/or ETDRS mapping information.

For example, as illustrated by FIG. 3, the segmented and annotated OCT B-scan 304 may be utilized to determine thickness measurements of one or more layers of the retina. The thickness measurements (e.g., numerical values) may be then mapped to the thickness map 306 in accordance with the ETDRS grid and/or ETDRS mapping information (e.g., in accordance with the nine subfields 308, 310, 312, 314, 316, 318, 320, 322, and 324 of the ETDRS grid). In this way, the thickness measurements may quantify the visual layer thickness as depicted by the thickness map 306. For example, as illustrated, the thickness measurements may include *J* microns mapped to subfield 308, *K* microns mapped to subfield 310, *L* microns mapped to subfield 312, *M* microns mapped to subfield 314, *N* microns mapped to subfield 316, *Q* microns mapped to subfield 318, *P* microns mapped to subfield 320, *R* microns mapped to subfield 322, and *S* microns mapped to subfield 324, where *J, K, L, M, N, Q, P, R,* and *S* each represents numerical values.

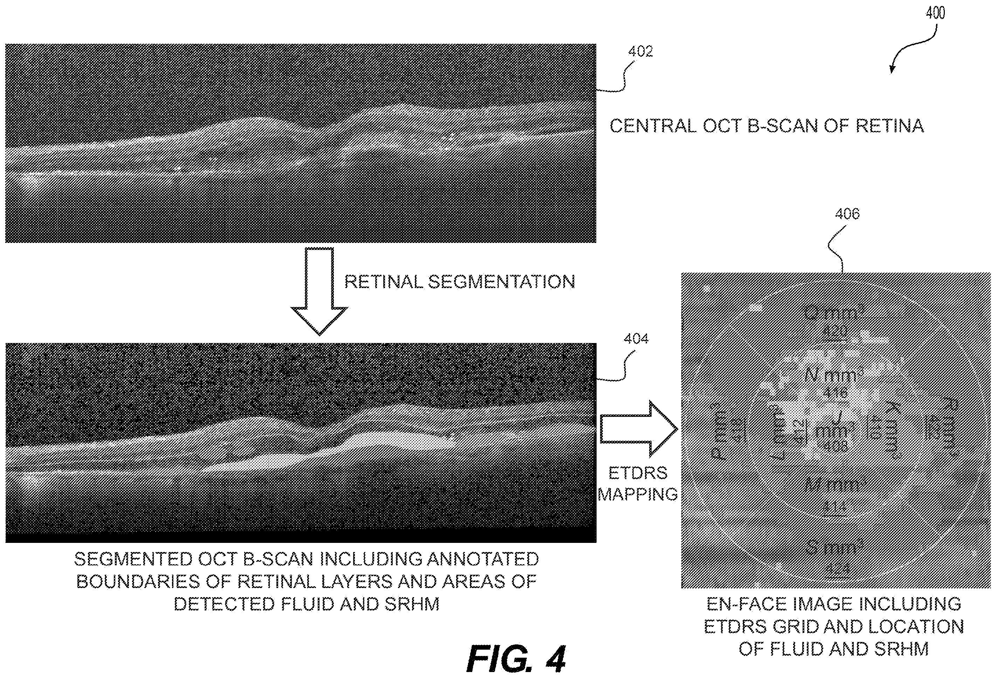

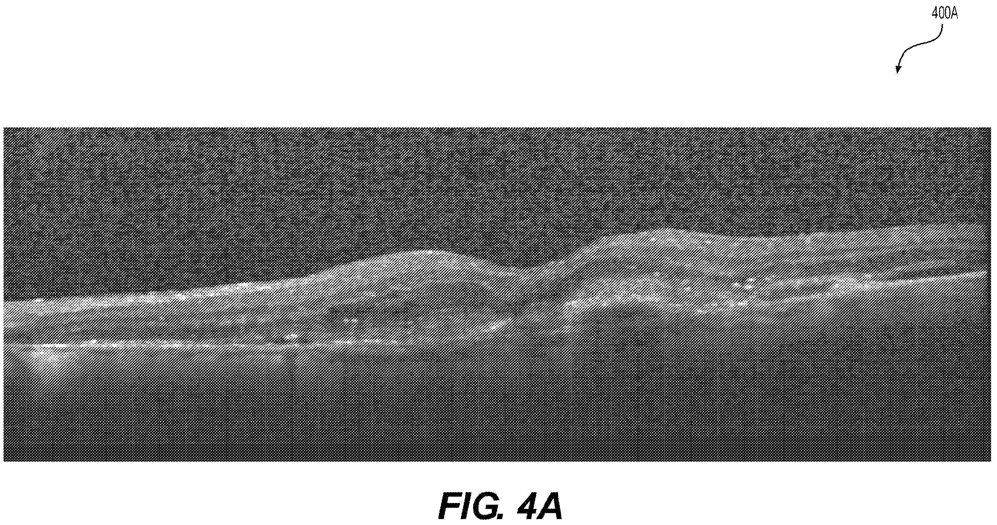

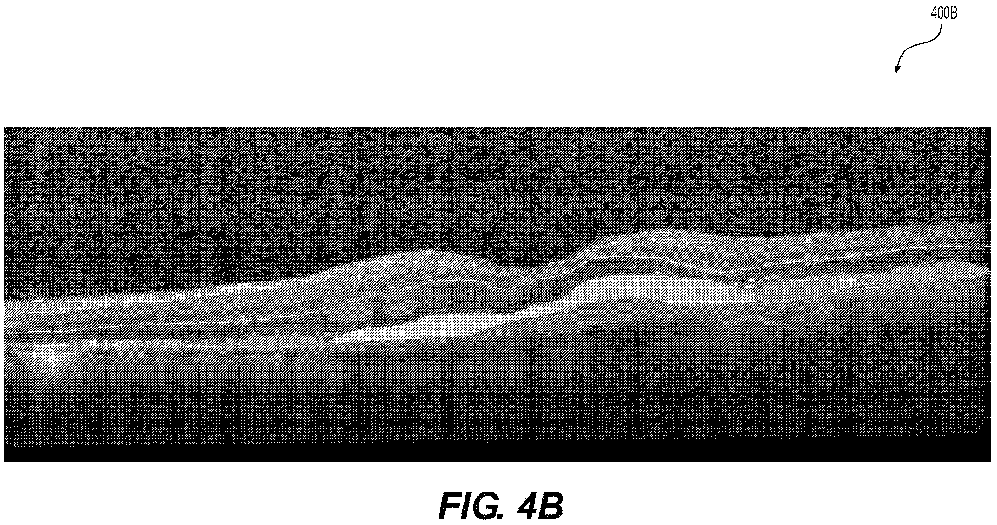

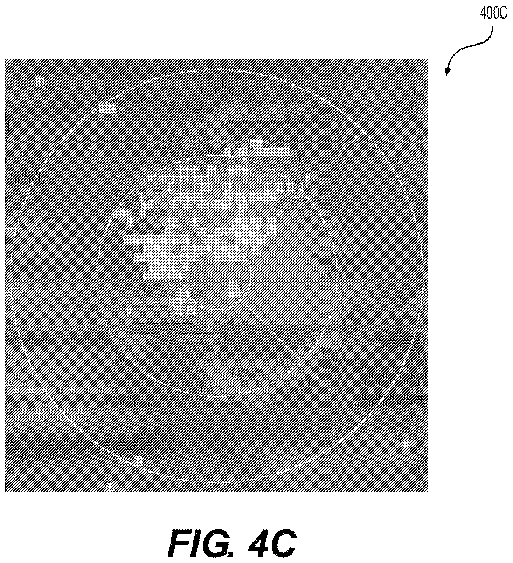

**FIGs. 4****,** **4A****,** **4B****,** and **4C** illustrate one or more additional high-resolution running examples 400, 400A, 400B, and 400C, respectively, for performing OCT retinal volumetric measurements based on the ETDRS grid and mapping the volumetric measurements and ETDRS grid to an en face retinal image, in accordance with the disclosed embodiments. The high-resolution running example 400 of FIG. 4 illustrates a central OCT B-scan 402 (e.g., corresponding to high-resolution central OCT B-scan 400A of FIG. 4A), a segmented and annotated OCT B-scan 404 (e.g., corresponding to high-resolution segmented and annotated OCT B-scan 400B of FIG. 4B), and an en face image 406 (e.g., corresponding to en face image 400C of FIG. 4C) illustrating one or more fluid and SRHM volume measurements. For example, referring to FIG. 4, the central OCT B-scan 402 may be an OCT B-scan generated based on one or more scans of patient's retina by an ophthalmoscope or other retinal image-capturing device. The central OCT B-scan 402 may be associated with the ETDRS grid and/or ETDRS mapping information.

As further illustrated by FIG. 4, the segmented and annotated OCT B-scan 404 may include annotations (e.g., colored lines) labeling one or more disease-associated features (e.g. fluid and SRHM) between one or more layers of the patient's retina. One or more retinal volumetric measurements (e.g., total volume measurements in cubic millimeters (mm³) of the fluid and SRHM or total volume in cubic microns (µm³) of the fluid and SRHM) may be then determined, and the one or more retinal volumetric measurements may be suitably mapped to the en face image 406 corresponding to the central OCT B-scan 402.

For example, as further illustrated by FIG. 4, the segmented and annotated OCT B-scan 404 may be utilized to determine volume measurements of the fluid and SRHM. The volume measurements (e.g., numerical values) may be then mapped to the en face image 406 in accordance with the ETDRS grid and/or ETDRS mapping information (e.g., in accordance with the nine subfields 408, 410, 412, 414, 416, 418, 420, 422, and 424 of the ETDRS grid). For example, as illustrated by the en face image 406, the volume measurements of the fluid and SRHM may include *J* mm³ mapped to subfield 408, *K* mm³ mapped to subfield 410, *L* mm³ mapped to subfield 412, *M* mm³ mapped to subfield 414, *N* mm³ mapped to subfield 416, *Q* mm³ mapped to subfield 418, *P* mm³ mapped to subfield 420, *R* mm³ mapped to subfield 422, and *S* mm³ mapped to subfield 424, where *J, K, L, M, N, Q, P, R,* and *S* each represents numerical values. In this way, the volume measurements of the fluid and SRHM may visually correspond to fluid and SRHM volume as displayed by the en face image 406.

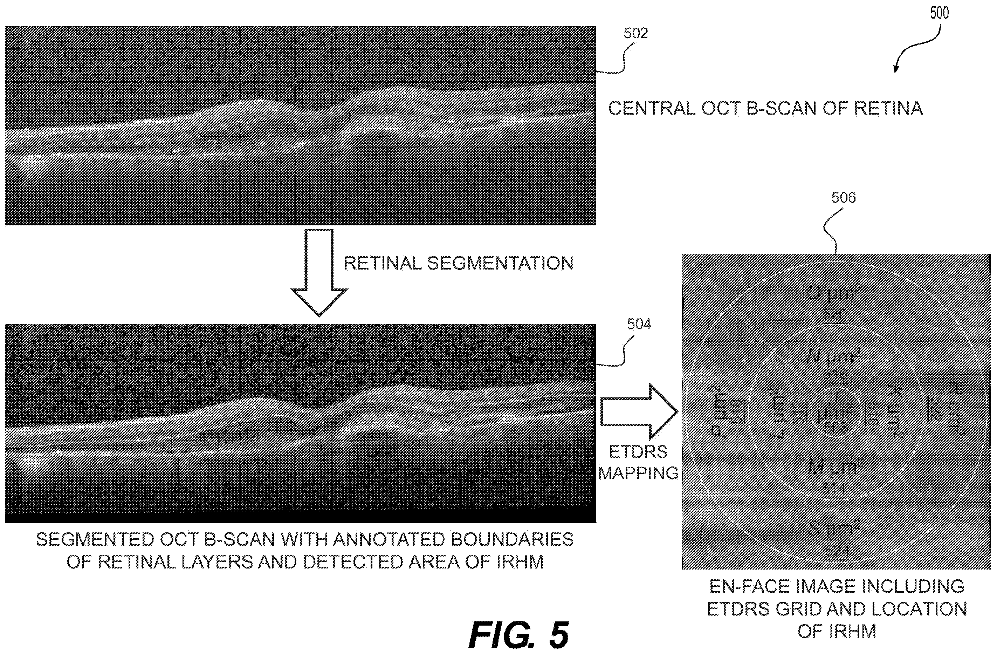

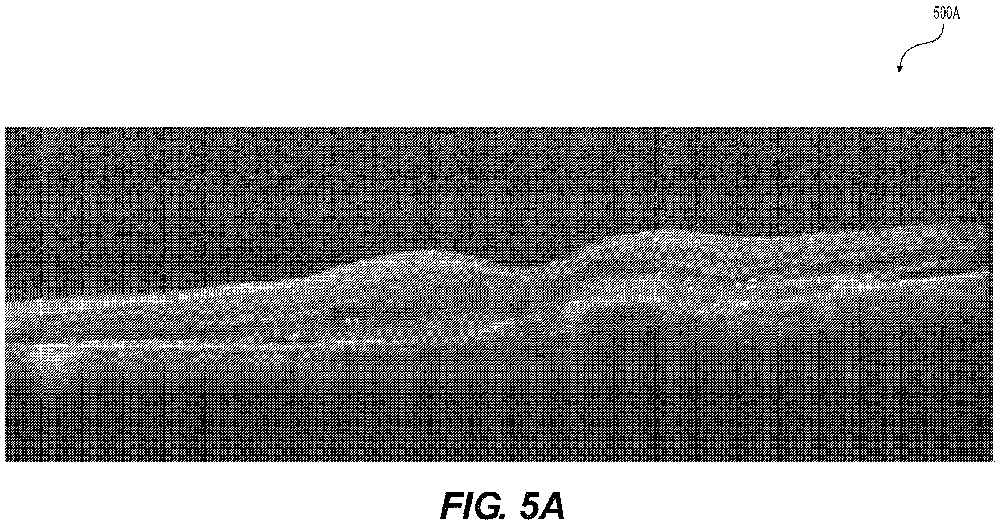

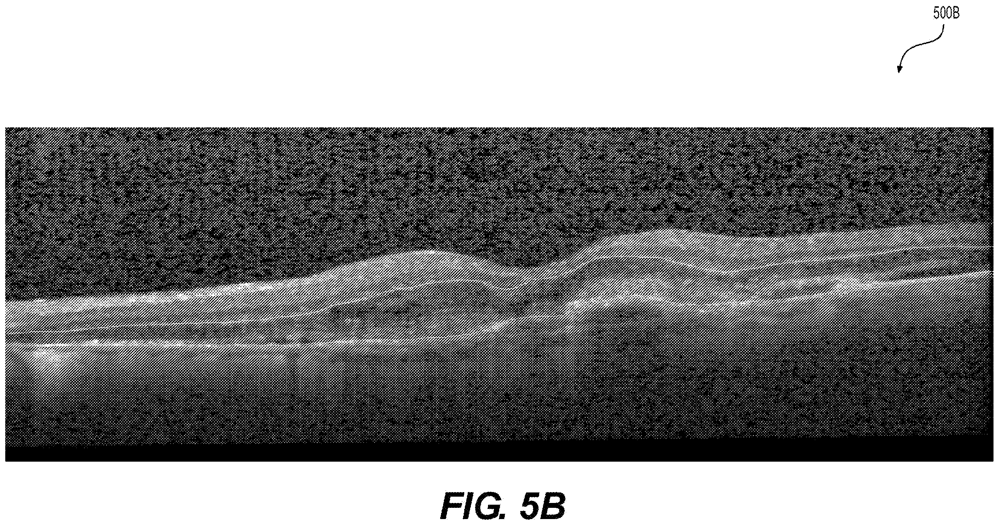

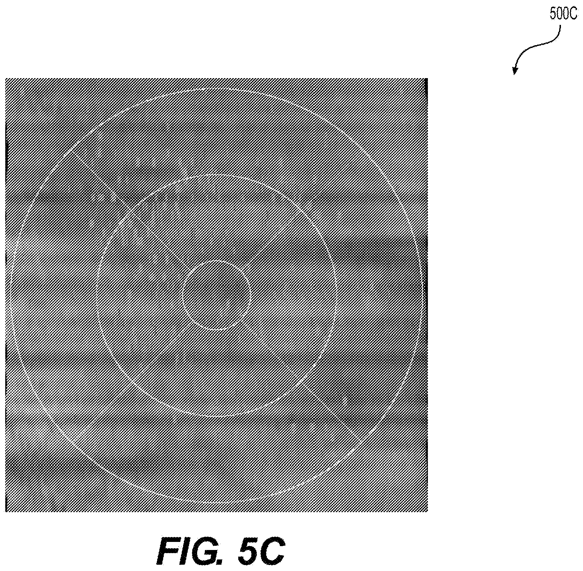

**FIGs. 5****,** **5A****,** **5B****,** and **5C** illustrate one or more additional high-resolution running examples 500, 500A, 500B, and 500C, respectively, for performing OCT retinal volumetric measurements based on the ETDRS grid and mapping the volumetric measurements and ETDRS grid to an en face retinal image, in accordance with the disclosed embodiments. The high-resolution running example 500 illustrates a central OCT B-scan 502 (e.g., corresponding to high-resolution central OCT B-scan 500A of FIG. 5A), a segmented and annotated OCT B-scan 504 (e.g., corresponding to high-resolution segmented and annotated OCT B-scan 500B of FIG. 5B), and an en face image 506 (e.g., corresponding to high-resolution en face image 500C of FIG. 5C) illustrating one or more IRHM area measurements. For example, referring to FIG. 5, the central OCT B-scan 502 may be an OCT B-scan generated based on one or more scans of patient's retina by an ophthalmoscope or other retinal image-capturing device. The central OCT B-scan 502 may be associated with the ETDRS grid and/or ETDRS mapping information.

As further illustrated by FIG. 5, the segmented and annotated OCT B-scan 504 may include annotations (e.g., colored lines) labeling one or more disease-associated features (e.g., IRHM) as deposited or dispersed among one or more layers of the patient's retina. One or more retinal volumetric measurements (e.g., area measurements in squared microns (µm²) of the deposit materials and/or area of disruption measurements in squared microns (µm²) of the deposit materials) may be determined, and the one or more area measurements may be suitably mapped to the en face image 506 corresponding to the central OCT B-scan 502.

For example, as further illustrated by FIG. 5, the segmented and annotated OCT B-scan 504 may be utilized to determine area measurements of the IRHM. The area measurements (e.g., numerical values) may be then mapped to the en face image 506 in accordance with the ETDRS grid and/or ETDRS mapping information (e.g., in accordance with the nine subfields 508, 510, 512, 514, 516, 518, 520, 522, and 524 of the ETDRS grid). For example, as illustrated by the en face image 506, the area measurements of the IRHM may include *J* µm² mapped to subfield 408, *K* µm² mapped to subfield 410, *L* µm² mapped to subfield 412, *M* µm² mapped to subfield 414, *N* µm² mapped to subfield 416, *Q* µm² mapped to subfield 418, *P* µm² mapped to subfield 420, *R* µm² mapped to subfield 422, and *S* µm² mapped to subfield 424, where *J, K, L, M, N, Q, P, R,* and *S* each represents numerical values. In this way, the area measurements of the IRHM may visually correspond to IRHM area as displayed by the en face image 506.

In certain embodiments, as may be appreciated with respect to the high-resolution thickness map 300C of FIG. 3C, the high-resolution en face retinal image 400C of FIG. 4C, and the high-resolution en face retinal image 500C of FIG. 5C, the ETDRS grid may be mapped to the one or more high-resolution retinal images 300C, 400C, and 500C, such that the nine subfields of the ETDRS grid may align visually with the visual features being displayed by the one or more high-resolution retinal images 300C, 400C, and 500C. In this way, the determined and reported retinal volumetric measurements (e.g., volume measurements, thickness measurements, area measurements, extent measurements, presence or absence indications, area of disruption measurements, and so forth) with respect the nine subfields of the ETDRS may correspond visually to the disease-associated features (e.g., layer thickness, fluid features, deposit material features) grid as displayed by the one or more high-resolution retinal images 300C, 400C, and 500C.

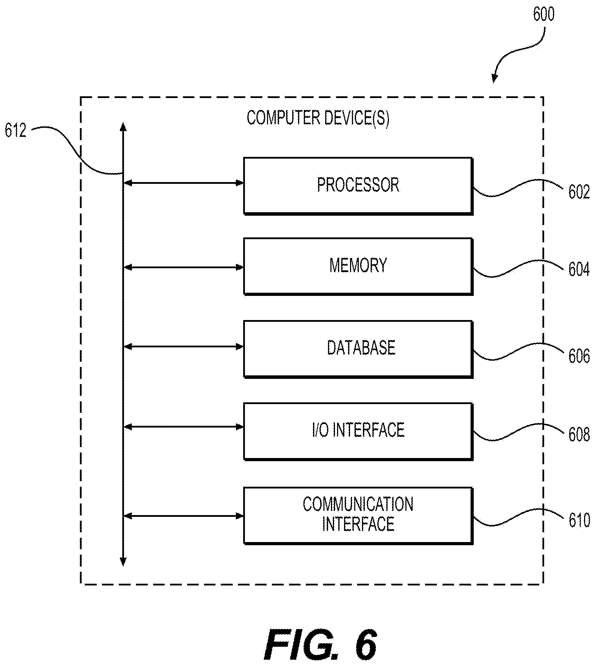

**FIG. 6** illustrates an example of one or more computing device(s) 600 that may be utilized for performing OCT retinal volumetric measurements based on the ETDRS grid and generating a clinical report based thereon, in accordance with the disclosed embodiments. In certain embodiments, the one or more computing device(s) 600 may perform one or more steps of one or more methods described or illustrated herein. In certain embodiments, the one or more computing device(s) 600 provide functionality described or illustrated herein. In certain embodiments, software running on the one or more computing device(s) 600 performs one or more steps of one or more methods described or illustrated herein, or provides functionality described or illustrated herein. Certain embodiments include one or more portions of the one or more computing device(s) 600.

This disclosure contemplates any suitable number of computing systems 600. This disclosure contemplates one or more computing device(s) 600 taking any suitable physical form. As example and not by way of limitation, one or more computing device(s) 600 may be an embedded computer system, a system-on-chip (SOC), a single-board computer system (SBC) (e.g., a computer-on-module (COM) or system-on-module (SOM)), a desktop computer system, a laptop or notebook computer system, an interactive kiosk, a mainframe, a mesh of computer systems, a mobile telephone, a personal digital assistant (PDA), a server, a tablet computer system, an augmented/virtual reality device, or a combination of two or more of these. Where appropriate, the one or more computing device(s) 600 may be unitary or distributed; span multiple locations; span multiple machines; span multiple data centers; or reside in a cloud, which may include one or more cloud components in one or more networks.

Where appropriate, the one or more computing device(s) 600 may perform without substantial spatial or temporal limitation one or more steps of one or more methods described or illustrated herein. As an example, and not by way of limitation, the one or more computing device(s) 600 may perform, in real-time or in batch mode, one or more steps of one or more methods described or illustrated herein. The one or more computing device(s) 600 may perform, at different times or at different locations, one or more steps of one or more methods described or illustrated herein, where appropriate.

In certain embodiments, the one or more computing device(s) 600 includes a processor 602, memory 604, database 606, an input/output (I/O) interface 608, a communication interface 610, and a bus 612. Although this disclosure describes and illustrates a particular computer system having a particular number of particular components in a particular arrangement, this disclosure contemplates any suitable computer system having any suitable number of any suitable components in any suitable arrangement. In certain embodiments, processor 602 includes hardware for executing instructions, such as those making up a computer program. As an example, and not by way of limitation, to execute instructions, processor 602 may retrieve (or fetch) the instructions from an internal register, an internal cache, memory 604, or database 606; decode and execute them; and then write one or more results to an internal register, an internal cache, memory 604, or database 606. In certain embodiments, processor 602 may include one or more internal caches for data, instructions, or addresses. This disclosure contemplates processor 602 including any suitable number of any suitable internal caches, where appropriate. As an example, and not by way of limitation, processor 602 may include one or more instruction caches, one or more data caches, and one or more translation lookaside buffers (TLBs). Instructions in the instruction caches may be copies of instructions in memory 604 or database 606, and the instruction caches may speed up retrieval of those instructions by processor 602.

Data in the data caches may be copies of data in memory 604 or database 606 for instructions executing at processor 602 to operate on; the results of previous instructions executed at processor 602 for access by subsequent instructions executing at processor 602 or for writing to memory 604 or database 606; or other suitable data. The data caches may speed up read or write operations by processor 602. The TLBs may speed up virtual-address translation for processor 602. In certain embodiments, processor 602 may include one or more internal registers for data, instructions, or addresses. This disclosure contemplates processor 602 including any suitable number of any suitable internal registers, where appropriate. Where appropriate, processor 602 may include one or more arithmetic logic units (ALUs); be a multicore processor; or include one or more processors 602. Although this disclosure describes and illustrates a particular processor, this disclosure contemplates any suitable processor.

In certain embodiments, memory 604 includes main memory for storing instructions for processor 602 to execute or data for processor 602 to operate on. As an example, and not by way of limitation, the one or more computing device(s) 600 may load instructions from database 606 or another source (such as, for example, another one or more computing device(s) 600) to memory 604. Processor 602 may then load the instructions from memory 604 to an internal register or internal cache. To execute the instructions, processor 602 may retrieve the instructions from the internal register or internal cache and decode them. During or after execution of the instructions, processor 602 may write one or more results (which may be intermediate or final results) to the internal register or internal cache. Processor 602 may then write one or more of those results to memory 604.

In certain embodiments, processor 602 executes only instructions in one or more internal registers, internal caches, or memory 604 (as opposed to database 606 or elsewhere) and operates only on data in one or more internal registers, internal caches, or memory 604 (as opposed to database 606 or elsewhere). One or more memory buses (which may each include an address bus and a data bus) may couple processor 602 to memory 604. Bus 612 may include one or more memory buses, as described below. In certain embodiments, one or more memory management units (MMUs) reside between processor 602 and memory 604 and facilitate accesses to memory 604 requested by processor 602. In certain embodiments, memory 604 includes random access memory (RAM). This RAM may be volatile memory, where appropriate. Where appropriate, this RAM may be dynamic RAM (DRAM) or static RAM (SRAM). Moreover, where appropriate, this RAM may be single-ported or multi-ported RAM. This disclosure contemplates any suitable RAM. Memory 604 may include one or more memory devices 604, where appropriate. Although this disclosure describes and illustrates particular memory, this disclosure contemplates any suitable memory.

In certain embodiments, database 606 includes mass storage for data or instructions. As an example, and not by way of limitation, database 606 may include a hard disk drive (HDD), a floppy disk drive, flash memory, an optical disc, a magneto-optical disc, magnetic tape, or a Universal Serial Bus (USB) drive, or a combination of two or more of these. Database 606 may include removable or non-removable (or fixed) media, where appropriate. Database 606 may be internal or external to the one or more computing device(s) 600, where appropriate. In certain embodiments, database 606 is non-volatile, solid-state memory. In certain embodiments, database 606 includes read-only memory (ROM). Where appropriate, this ROM may be mask-programmed ROM, programmable ROM (PROM), erasable PROM (EPROM), electrically erasable PROM (EEPROM), electrically alterable ROM (EAROM), flash memory, or a combination of two or more of these. This disclosure contemplates mass database 606 taking any suitable physical form. Database 606 may include one or more storage control units facilitating communication between processor 602 and database 606, where appropriate. Where appropriate, database 606 may include one or more databases 606. Although this disclosure describes and illustrates particular storage, this disclosure contemplates any suitable storage.

In certain embodiments, I/O interface 608 includes hardware, software, or both, providing one or more interfaces for communication between the one or more computing device(s) 600 and one or more I/O devices. The one or more computing device(s) 600 may include one or more of these I/O devices, where appropriate. One or more of these I/O devices may enable communication between a person and the one or more computing device(s) 600. As an example, and not by way of limitation, an I/O device may include a keyboard, keypad, microphone, monitor, mouse, printer, scanner, speaker, still camera, stylus, tablet, touch screen, trackball, video camera, another suitable I/O device, or a combination of two or more of these. An I/O device may include one or more sensors. This disclosure contemplates any suitable I/O devices and any suitable I/O interfaces 608 for them. Where appropriate, I/O interface 608 may include one or more device or software drivers enabling processor 602 to drive one or more of these I/O devices. I/O interface 608 may include one or more I/O interfaces 608, where appropriate. Although this disclosure describes and illustrates a particular I/O interface, this disclosure contemplates any suitable I/O interface.

In certain embodiments, communication interface 610 includes hardware, software, or both providing one or more interfaces for communication (such as, for example, packet-based communication) between the one or more computing device(s) 600 and one or more other computing device(s) 600 or one or more networks. As an example, and not by way of limitation, communication interface 610 may include a network interface controller (NIC) or network adapter for communicating with an Ethernet or other wire-based network or a wireless NIC (WNIC) or wireless adapter for communicating with a wireless network, such as a WI-FI network. This disclosure contemplates any suitable network and any suitable communication interface 610 for it.

As an example, and not by way of limitation, the one or more computing device(s) 600 may communicate with an ad hoc network, a personal area network (PAN), a local area network (LAN), a wide area network (WAN), a metropolitan area network (MAN), one or more portions of the Internet, or a combination of two or more of these. One or more portions of one or more of these networks may be wired or wireless. As an example, the one or more computing device(s) 600 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), other suitable wireless network, or a combination of two or more of these. The one or more computing device(s) 600 may include any suitable communication interface 610 for any of these networks, where appropriate. Communication interface 610 may include one or more communication interfaces 610, where appropriate. Although this disclosure describes and illustrates a particular communication interface, this disclosure contemplates any suitable communication interface.

In certain embodiments, bus 612 includes hardware, software, or both coupling components of the one or more computing device(s) 600 to each other. As an example, and not by way of limitation, bus 612 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, another suitable bus, or a combination of two or more of these. Bus 612 may include one or more buses 612, where appropriate. Although this disclosure describes and illustrates a particular bus, this disclosure contemplates any suitable bus or interconnect.

Herein, a computer-readable non-transitory storage medium or media may include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs, optical disc drives (ODDs), magneto-optical discs, magneto-optical drives, floppy diskettes, floppy disk drives (FDDs), magnetic tapes, solid-state drives (SSDs), RAM-drives, SECURE DIGITAL cards or drives, any other suitable computer-readable non-transitory storage media, or any suitable combination of two or more of these, where appropriate. A computer-readable non-transitory storage medium may be volatile, non-volatile, or a combination of volatile and non-volatile, where appropriate.

Herein, "or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

Herein, "automatically" and its derivatives means "without human intervention," unless expressly indicated otherwise or indicated otherwise by context.

The embodiments disclosed herein are only examples, and the scope of this disclosure is not limited to them. Embodiments according to this disclosure are in particular disclosed in the attached claims directed to a method, a storage medium, a system and a computer program product, wherein any feature mentioned in one claim category, e.g., method, may be claimed in another claim category, e.g., system, as well. The dependencies or references back in the attached claims are chosen for formal reasons only. However, any subject matter resulting from a deliberate reference back to any previous claims (in particular multiple dependencies) may be claimed as well, so that any combination of claims and the features thereof are disclosed and may be claimed regardless of the dependencies chosen in the attached claims. The subject matter which may be claimed comprises not only the combinations of features as set out in the attached claims but also any other combination of features in the claims, wherein each feature mentioned in the claims may be combined with any other feature or combination of other features in the claims. Furthermore, any of the embodiments and features described or depicted herein may be claimed in a separate claim and/or in any combination with any embodiment or feature described or depicted herein or with any of the features of the attached claims.

The scope of this disclosure encompasses all changes, substitutions, variations, alterations, and modifications to the example embodiments described or illustrated herein that a person having ordinary skill in the art would comprehend. The scope of this disclosure is not limited to the example embodiments described or illustrated herein. Moreover, although this disclosure describes and illustrates respective embodiments herein as including particular components, elements, feature, functions, operations, or steps, any of these embodiments may include any combination or permutation of any of the components, elements, features, functions, operations, or steps described or illustrated anywhere herein that a person having ordinary skill in the art would comprehend. Furthermore, reference in the appended claims to an apparatus or system or a component of an apparatus or system being adapted to, arranged to, capable of, configured to, enabled to, operable to, or operative to perform a particular function encompasses that apparatus, system, component, whether or not it or that particular function is activated, turned on, or unlocked, as long as that apparatus, system, or component is so adapted, arranged, capable, configured, enabled, operable, or operative. Additionally, although this disclosure describes or illustrates certain embodiments as providing particular advantages, certain embodiments may provide none, some, or all of these advantages.

## Claims

1. A computer implemented method for performing retinal volumetric measurements based on the Early Treatment for Diabetic Retinopathy Study (ETDRS) grid, by one or more computing devices:
receiving an optical coherence tomography (OCT) image of a retina of a patient and ETDRS mapping information identifying one or more subfields of the ETDRS grid;
segmenting the OCT image of the retina to identify one or more layer features corresponding to layers of the retina;
segmenting the OCT image of the retina to obtain an initial feature-segmented OCT image comprising one or more disease-associated features associated with the one or more layer features;
refining the one or more disease-associated features by generating, based on the layer features and the initial feature-segmented image, a refined segmented OCT image;
determining, based on the refined segmented OCT image, one or more volumetric measurements of the one or more refined disease-associated features, wherein the one or more volumetric measurements correspond to the ETDRS mapping information; and
generating a report based on the one or more volumetric measurements.

2. The method of Claim 1, wherein the one or more layer features comprise a Bruch's membrane (BM), a boundary of myoid and ellipsoid inner segments (BMEIS), a ganglion cell layer-inner plexiform layer (GCL-IPL), an inner boundary outer photoreceptor (IB-OPR) layer, an outer boundary outer photoreceptor (OB-OPR) layer, an inner boundary retinal pigment epithelium (IB-RPE) layer, an outer boundary retinal pigment epithelium (OB-RPE) layer, an internal limiting membrane (ILM), an inner plexiform layer-inner nuclear layer (IPL-INL), an inner plexiform layer-outer nuclear layer (IPL-ONL), an inner segment/outer segment junction (ISJ-OSJ) layer, outer plexiform layer-Henle's fiber layer (OPL-HFL), or a retinal nerve fiber layer-ganglion cell layer (RNFL-GCL).

3. The method of Claim 1, wherein the one or more disease-associated features comprises one or more fluid features or one or more deposit features, the one or more fluid features comprising one or more of an intraretinal fluid (IRF), a subretinal fluid (SRF), or a fluid corresponding to pigment epithelial detachment (PED), and/or the one or more deposit features comprising a subretinal hyperreflective material (SHRM), an intraretinal hyperreflective material (IHRM), or a hyperreflective retinal foci (HRF).

4. The method of Claim 1, further comprising identifying one or more biomarkers based on the one or more volumetric measurements.

5. The method of Claim 1, further comprising:
receiving an en face image of the retina of the patient, wherein the en face image is associated with the OCT image; and
prior to generating the report, mapping the one or more volumetric measurements and the ETDRS mapping information to the en face image.

6. The method of Claim 1, wherein determining the one or more volumetric measurements comprises determining a total volume of the one or more disease-associated features with respect to at least one of the one or more identified subfields, determining a fluid volume of one or more fluid features with respect to at least one of the one or more identified subfields or determining a deposit volume of one or more deposit features with respect to at least one of the one or more identified subfields.

7. The method of Claim 1, wherein determining the one or more volumetric measurements comprises determining a fluid extent of the one or more fluid features with respect to at least one of the one or more identified subfields.

8. The method of Claim 1, wherein determining the one or more volumetric measurements comprises determining a number of one or more deposit features with respect to at least one of the one or more identified subfields.

9. The method of Claim 1, wherein determining the one or more volumetric measurements comprises determining an area of the one or more disease-associated features with respect to at least one of the one or more identified subfields, determining a presence or an absence of the one or more disease-associated features with respect to at least one of the one or more identified subfields, or determining an area of disruption of the one or more disease-associated features with respect to at least one of the one or more identified subfields.

10. The method of Claim 1, wherein the OCT image comprises a time-domain optical coherence tomography (TD-OCT) image or a spectral-domain optical coherence tomography (SD-OCT) image.

11. The method of Claim 1, wherein the OCT image comprises an image of a fovea of the patient captured by an OCT ophthalmoscope, and wherein the image of the fovea was further divided into three concentric circles with diameters of approximately 1 millimeter (mm), approximately 3mm, and approximately 6mm, respectively, in accordance with the ETDRS grid.

12. The method of Claim 1, further comprising:
receiving an optical coherence tomography angiography (OCT-A) image of the retina of the patient; and
generating a retinal vascular 3D map of the retina based on the OCT-A image and the one or more volumetric measurements.

13. The method of Claim 1, further comprising:
receiving a color fundus photography (CFP) image of the retina of the patient and
generating a composite image of the retina based on the CFP image and the one or more volumetric measurements.

14. A system for performing retinal volumetric measurements based on the Early Treatment for Diabetic Retinopathy Study (ETDRS) grid, the system including one or more computing devices, comprising:
one or more non-transitory computer-readable storage media including instructions; and
one or more processors coupled to the one or more storage media, the one or more processors configured to execute the instructions to:
receive an optical coherence tomography (OCT) image of a retina of a patient and ETDRS mapping information identifying one or more subfields of the ETDRS grid;
segment the OCT image of the retina to identify one or more layer features of the retina;
segment the OCT image of the retina to obtain an initial feature-segmented OCT image comprising one or more disease-associated features;
refine the one or more disease-associated features by generating, based on the layer features and the initial feature-segmented image, a refined segmented OCT image;
determine, based on the refined segmented OCT image, one or more volumetric measurements of the one or more refined disease-associated features associated with at least one of the layer features and corresponding to the ETDRS mapping information; and
generate a report based on the one or more volumetric measurements.

## Patentansprüche

1. Computerimplementiertes Verfahren zur Durchführung volumetrischer Retinamessungen basierend auf dem Gitter der Early Treatment for Diabetic Retinopathy Study (ETDRS) durch eine oder mehrere Rechenvorrichtungen:
Empfangen eines optischen Kohärenztomographie-(OCT-)Bildes einer Retina eines Patienten und von ETDRS-Abbildungsinformationen, die ein oder mehrere Teilfelder des ETDRS-Gitters identifizieren;
Segmentieren des OCT-Bildes der Retina, um ein oder mehrere Schichtmerkmale zu identifizieren, die Schichten der Retina entsprechen;
Segmentieren des OCT-Bildes der Retina, um ein anfängliches merkmalssegmentiertes OCT-Bild zu erhalten, das ein oder mehrere krankheitsassoziierte Merkmale umfasst, die mit dem einen oder den mehreren Schichtmerkmalen assoziiert sind;
Verfeinern des einen oder der mehreren krankheitsassoziierten Merkmale durch Erzeugen eines verfeinerten segmentierten OCT-Bildes basierend auf den Schichtmerkmalen und dem anfänglichen merkmalssegmentierten Bild;
Bestimmen einer oder mehrerer volumetrischer Messungen des einen oder der mehreren verfeinerten krankheitsassoziierten Merkmale basierend auf dem verfeinerten segmentierten OCT-Bild, wobei die eine oder die mehreren volumetrischen Messungen den ETDRS-Abbildungsinformationen entsprechen; und
Erzeugen eines Berichts basierend auf der einen oder den mehreren volumetrischen Messungen.

2. Verfahren nach Anspruch 1, wobei das eine oder die mehreren Schichtmerkmale eine Bruch'sche Membran (BM), eine Grenze von myoiden und ellipsoiden inneren Segmenten (BMEIS), eine Ganglienzellschicht/innere plexiforme Schicht (GCL-IPL), eine innere Grenze der äußeren Photorezeptorschicht (IB-OPR), eine äußere Grenze der äußeren Photorezeptorschicht (OB-OPR), eine innere Grenze der retinalen Pigmentepithelschicht (IB-RPE), eine äußere Grenze der retinalen Pigmentepithelschicht (OB-RPE), eine interne begrenzende Membran (ILM), eine innere plexiforme Schicht/innere Kernschicht (IPL-INL), eine innere plexiforme Schicht/äußere Kernschicht (IPL-ONL), eine innere Segment/äußere Segment-Übergangsschicht (ISJ-OSJ), äußere plexiforme Schicht/Henle'sche Faserschicht (OPL-HFL) oder eine retinale Nervenfaserschicht/Ganglienzellschicht (RNFL-GCL) umfassen.

3. Verfahren nach Anspruch 1, wobei das eine oder die mehreren krankheitsbedingten Merkmale ein oder mehrere Fluidmerkmale oder ein oder mehrere Ablagerungsmerkmale umfassen, wobei das eine oder die mehreren Fluidmerkmale eines oder mehrere von einem intraretinalen Fluid (IRF), einem subretinalen Fluid (SRF) oder einem Fluid, das Pigmentepithelablösung (PED) entspricht, umfassen und/oder das eine oder die mehreren Ablagerungsmerkmale ein subretinales hyperreflektierendes Material (SHRM), ein intraretinales hyperreflektierendes Material (IHRM) oder einen hyperreflektierenden retinalen Fokus (HRF) umfassen.

4. Verfahren nach Anspruch 1, ferner umfassend Identifizieren eines oder mehrerer Biomarker basierend auf der einen oder den mehreren volumetrischen Messungen.

5. Verfahren nach Anspruch 1, ferner umfassend:
Empfangen eines En-Face-Bildes der Retina des Patienten, wobei das En-Face-Bild mit dem OCT-Bild assoziiert ist; und
Abbilden der einen oder der mehreren volumetrischen Messungen und der ETDRS-Abbildungsinformationen auf das En-Face-Bild vor dem Erzeugen des Berichts.

6. Verfahren nach Anspruch 1, wobei das Bestimmen der einen oder der mehreren volumetrischen Messungen Bestimmen eines Gesamtvolumens des einen oder der mehreren krankheitsassoziierten Merkmale in Bezug auf mindestens eines des einen oder der mehreren identifizierten Teilfelder, Bestimmen eines Fluidvolumens eines oder mehrerer Fluidmerkmale in Bezug auf mindestens eines des einen oder der mehreren identifizierten Teilfelder oder Bestimmen eines Ablagerungsvolumens eines oder mehrerer Ablagerungsmerkmale in Bezug auf mindestens eines des einen oder der mehreren identifizierten Teilfelder umfasst.

7. Verfahren nach Anspruch 1, wobei das Bestimmen der einen oder der mehreren volumetrischen Messungen Bestimmen eines Fluidausmaßes des einen oder der mehreren Fluidmerkmale in Bezug auf mindestens eines des einen oder der mehreren identifizierten Teilfelder umfasst.

8. Verfahren nach Anspruch 1, wobei das Bestimmen der einen oder der mehreren volumetrischen Messungen Bestimmen einer Anzahl von einem oder mehreren Ablagerungsmerkmalen in Bezug auf mindestens eines des einen oder der mehreren identifizierten Teilfelder umfasst.

9. Verfahren nach Anspruch 1, wobei das Bestimmen der einen oder der mehreren volumetrischen Messungen Bestimmen eines Bereichs des einen oder der mehreren krankheitsassoziierten Merkmale in Bezug auf mindestens eines des einen oder der mehreren identifizierten Teilfelder, Bestimmen eines Vorhandenseins oder einer Abwesenheit des einen oder der mehreren krankheitsassoziierten Merkmale in Bezug auf mindestens eines des einen oder der mehreren identifizierten Teilfelder oder Bestimmen eines Störungsbereichs des einen oder der mehreren krankheitsassoziierten Merkmale in Bezug auf mindestens eines des einen oder der mehreren identifizierten Teilfelder umfasst.

10. Verfahren nach Anspruch 1, wobei das OCT-Bild ein optisches Zeitdomänen-Kohärenztomographie-(TD-OCT-)Bild oder ein optisches Spektraldomänen-Kohärenztomographie-(SD-OCT-)Bild umfasst.

11. Verfahren nach Anspruch 1, wobei das OCT-Bild ein Bild einer Fovea des Patienten umfasst, das von einem OCT-Ophthalmoskop aufgenommen wurde, und wobei das Bild der Fovea ferner in drei konzentrische Kreise mit Durchmessern von ungefähr 1 Millimeter (mm), ungefähr 3 mm bzw. ungefähr 6 mm gemäß dem ETDRS-Gitter unterteilt wurde.

12. Verfahren nach Anspruch 1, ferner umfassend:
Empfangen eines optischen Kohärenztomographie-Angiographie-(OCT-A-)Bildes der Retina des Patienten; und
Erzeugen einer retinalen vaskulären 3D-Abbildung der Retina basierend auf dem OCT-A-Bild und der einen oder den mehreren volumetrischen Messungen.

13. Verfahren nach Anspruch 1, ferner umfassend:
Empfangen eines Farbfundusfotografie-(CFP-)Bildes der Retina des Patienten und Erzeugen eines zusammengesetzten Bildes der Retina basierend auf dem CFP-Bild und der einen oder den mehreren volumetrischen Messungen.

14. System zur Durchführung volumetrischer Retinamessungen basierend auf dem Gitter der Early Treatment for Diabetic Retinopathy Study (ETDRS), wobei das System eine oder mehrere Rechenvorrichtungen beinhaltet, umfassend:
ein oder mehrere nichtflüchtige computerlesbare Speichermedien, die Anweisungen beinhalten; und
einen oder mehrere Prozessoren, die mit dem einen oder den mehreren Speichermedien gekoppelt sind, wobei der eine oder die mehreren Prozessoren konfiguriert sind, um die Anweisungen zu Folgendem auszuführen:
Empfangen eines optischen Kohärenztomographie-(OCT-)Bildes einer Retina eines Patienten und von ETDRS-Abbildungsinformationen, die ein oder mehrere Teilfelder des ETDRS-Gitters identifizieren;
Segmentieren des OCT-Bildes der Retina, um ein oder mehrere Schichtmerkmale der Retina zu identifizieren;
Segmentieren des OCT-Bildes der Retina, um ein anfängliches merkmalssegmentiertes OCT-Bild zu erhalten, das ein oder mehrere krankheitsassoziierte Merkmale umfasst;
Verfeinern des einen oder der mehreren krankheitsassoziierten Merkmale durch Erzeugen eines verfeinerten segmentierten OCT-Bildes basierend auf den Schichtmerkmalen und dem anfänglichen merkmalssegmentierten Bild;
Bestimmen einer oder mehrerer volumetrischer Messungen des einen oder der mehreren verfeinerten krankheitsassoziierten Merkmale, die mit mindestens einem der Schichtmerkmale assoziiert sind und den ETDRS-Abbildungsinformationen entsprechen, basierend auf dem verfeinerten segmentierten OCT-Bild; und
Erzeugen eines Berichts basierend auf der einen oder den mehreren volumetrischen Messungen.

## Revendications

1. Procédé mis en œuvre par ordinateur pour réaliser des mesures volumétriques rétiniennes basées sur la grille d'étude du traitement précoce de la rétinopathie diabétique (ETDRS), par un ou plusieurs dispositifs de calcul :
réception d'une image de tomographie en cohérence optique (OCT) d'une rétine d'un patient et des informations de mappage d'étude ETDRS identifiant un ou plusieurs sous-champs de la grille d'étude ETDRS ;
segmentation de l'image OCT de la rétine pour identifier une ou plusieurs caractéristiques de couche correspondant à des couches de la rétine ;
segmentation de l'image OCT de la rétine pour obtenir une image OCT segmentée initiale selon les caractéristiques comprenant une ou plusieurs caractéristiques associées à une maladie associées à la ou aux caractéristiques de couche ;
affinement de la ou des caractéristiques associées à une maladie en générant, en se basant sur les caractéristiques de couche et l'image segmentée initiale selon les caractéristiques, une image OCT segmentée affinée ;
détermination, en se basant sur l'image OCT segmentée affinée, d'une ou de plusieurs mesures volumétriques de la ou des caractéristiques affinées associées à une maladie, dans lequel la ou les mesures volumétriques correspondent aux informations de mappage d'étude ETDRS ; et
génération d'un rapport basé sur la ou les mesures volumétriques.

2. Procédé selon la revendication 1, dans lequel la ou les caractéristiques de couche comprennent une membrane de Bruch (BM), une limite des segments internes myoïdes et ellipsoïdes (BMEIS), une couche des cellules ganglionnaires-couche plexiforme interne (GCL-IPL), une couche limite interne des photorécepteurs externes (IB-OPR), une couche limite externe des photorécepteurs externes (OB-OPR), une couche limite interne de l'épithélium pigmentaire rétinien (IB-RPE), une couche limite externe de l'épithélium pigmentaire rétinien (OB-RPE), une membrane limitante interne (MLI), une couche plexiforme interne-couche nucléaire interne (IPL-INL), une couche plexiforme interne-couche nucléaire externe (IPL-ONL), une couche de jonction segment interne/segment externe (ISJ-OSJ), une couche plexiforme externe-couche des fibres de Henle (OPL-HFL) ou une couche de fibres nerveuses rétiniennes-couche de cellules ganglionnaires (RNFL-GCL).

3. Procédé selon la revendication 1, dans lequel la ou les caractéristiques associées à une maladie comprennent une ou plusieurs caractéristiques de liquide ou une ou plusieurs caractéristiques de dépôt, la ou les caractéristiques de liquide comprenant un ou plusieurs parmi un liquide intrarétinien (LIR), un liquide sous-rétinien (LSR) ou un liquide correspondant à un décollement de l'épithélium pigmentaire (DEP), et/ou la ou les caractéristiques de dépôt comprenant un matériel hyperréflectif sous-rétinien (SHRM), un matériel hyperréflectif intrarétinien (IHRM) ou des foyers rétiniens hyperréflectifs (HRF).

4. Procédé selon la revendication 1, comprenant en outre l'identification d'un ou de plusieurs biomarqueurs basés sur la ou les mesures volumétriques.

5. Procédé selon la revendication 1, comprenant en outre :
la réception d'une image en face de la rétine du patient, dans lequel l'image en face est associée à l'image OCT ; et
avant la génération du rapport, le mappage de la ou des mesures volumétriques et des informations de mappage d'étude ETDRS sur l'image en face.

6. Procédé selon la revendication 1, dans lequel la détermination de la ou des mesures volumétriques comprend la détermination d'un volume total de la ou des caractéristiques associées à une maladie par rapport à au moins un du ou des sous-champs identifiés, la détermination d'un volume de liquide d'une ou de plusieurs caractéristiques de liquide par rapport à au moins un du ou des sous-champs identifiés ou la détermination d'un volume de dépôt d'une ou de plusieurs caractéristiques de dépôt par rapport à au moins un du ou des sous-champs identifiés.

7. Procédé selon la revendication 1, dans lequel la détermination de la ou des mesures volumétriques comprend la détermination d'un niveau de liquide de la ou des caractéristiques de liquide par rapport à au moins un du ou des sous-champs identifiés.

8. Procédé selon la revendication 1, dans lequel la détermination de la ou des mesures volumétriques comprend la détermination d'un nombre d'une ou de plusieurs caractéristiques de dépôt par rapport à au moins un du ou des sous-champs identifiés.

9. Procédé selon la revendication 1, dans lequel la détermination de la ou des mesures volumétriques comprend la détermination d'une aire de la ou des caractéristiques associées à une maladie par rapport à au moins un du ou des sous-champs identifiés, la détermination d'une présence ou d'une absence de la ou des caractéristiques associées à une maladie par rapport à au moins un du ou des sous-champs identifiés, ou la détermination d'une aire de perturbation de la ou des caractéristiques associées à une maladie par rapport à au moins un du ou des sous-champs identifiés.

10. Procédé selon la revendication 1, dans lequel l'image OCT comprend une image de tomographie en cohérence optique dans le domaine temporel (TD-OCT) ou une image de tomographie en cohérence optique dans le domaine spectral (SD-OCT).

11. Procédé selon la revendication 1, dans lequel l'image OCT comprend une image d'une fovéa du patient capturée par un ophtalmoscope OCT, et dans lequel l'image de la fovéa a en outre été divisée en trois cercles concentriques avec des diamètres d'environ 1 millimètre (mm), d'environ 3 mm et d'environ 6 mm, respectivement, conformément à la grille d'étude ETDRS.

12. Procédé selon la revendication 1, comprenant en outre :
la réception d'une image de tomographie en cohérence optique-angiographie (OCT-A) de la rétine du patient ; et
la génération d'une carte vasculaire rétinienne 3D de la rétine basée sur l'image OCT-A et la ou les mesures volumétriques.

13. Procédé selon la revendication 1, comprenant en outre :
la réception d'une image de photographie de fond d'œil en couleur (CFP) de la rétine du patient et la génération d'une image composite de la rétine basée sur l'image CFP et la ou les mesures volumétriques.

14. Système de réalisation de mesures volumétriques rétiniennes basées sur la grille d'étude du traitement précoce de la rétinopathie diabétique (ETDRS), le système comprenant un ou plusieurs dispositifs de calcul, comprenant :
un ou plusieurs supports de stockage lisibles par ordinateur non transitoires comprenant des instructions ; et
un ou plusieurs processeurs couplés au ou aux supports de stockage, le ou les processeurs étant configurés pour exécuter les instructions afin de :
recevoir une image de tomographie en cohérence optique (OCT) d'une rétine d'un patient et des informations de mappage d'étude ETDRS identifiant un ou plusieurs sous-champs de la grille d'étude ETDRS ;
segmenter l'image OCT de la rétine pour identifier une ou plusieurs caractéristiques de couche de la rétine ;
segmenter l'image OCT de la rétine pour obtenir une image OCT segmentée initiale selon les caractéristiques comprenant une ou plusieurs caractéristiques associées à une maladie ;
affiner la ou les caractéristiques associées à une maladie en générant, en se basant sur les caractéristiques de couche et l'image segmentée initiale selon les caractéristiques, une image OCT segmentée affinée ;
déterminer, en se basant sur l'image OCT segmentée affinée, une ou plusieurs mesures volumétriques de la ou des caractéristiques associées à une maladie affinées associées à au moins une des caractéristiques de couche et correspondant aux informations de mappage d'étude ETDRS ; et
générer un rapport basé sur la ou les mesures volumétriques.
